# EUROPEAN PATENT SPECIFICATION

(11) **EP 4 028 704 B1**
(45) Date of publication and mention of the grant of the patent: **03.04.2024**
(21) Application number: 20807103.5
(22) Date of filing: 14.09.2020
(51) Int. Cl.: F26B 3/00, B01J 20/26, B01J 20/28, B01J 20/291, C07K 1/16, F26B 21/14

(54) **SUPERCRITICAL DRYING OF CHROMATOGRAPHIC MEDIA**
ÜBERKRITISCHE TROCKNUNG VON CHROMATOGRAPHISCHEN MEDIEN
SÉCHAGE SUPERCRITIQUE DES MILIEUX CHROMATOGRAPHIQUES

(30) Priority: 13.09.2019 US 201962900155 P; 16.09.2019 US 201962900798 P
(43) Date of publication of application: 20.07.2022
(73) Proprietor: Merck Millipore Ltd., Carrigtwohill (IE)
(72) Inventor: BOYLE, John, Burlington, MA 01803 (US); SKARJA, Gary, Burlington, MA 01803 (US); RAGHEB, Amro, Burlington, MA 01803 (US)
(74) Representative: HGF
(86) International application number: PCT/IB2020/000832
(87) International publication number: WO 2021/048633

(56) References cited:
- WO-A2-2014/140860
- CN-A- 101 851 398
- GB-A- 2 571 292
- US-A1- 2002 187 302
- US-A1- 2005 079 337
- US-A1- 2011 253 616
- US-A1- 2012 231 251
- US-A1- 2016 243 538
- US-A1- 2019 100 630
- US-B1- 6 638 885

## Description

### RELATED APPLICATIONS

This application claims the benefit of priority to US Provisional Patent Application No. 62/900,798, filed September 16, 2019; and US Provisional Patent Application No. 62/900,155, filed September 13, 2019.

### BACKGROUND

Supercritical fluid (SCF) technology has found many uses in variety of fields, especially in pharmaceutical applications, such as particles and crystal engineering, where great control over the physicochemical properties of the produced particles and control of crystal formation is highly desirable. Other applications, such as composite particle formation, extraction, coating, and preparation of liposomes, have found deep interest in SCF technology.

The use of critical point drying (CPD) has also generated interest in several fields that require the preservation of structure during drying, such as drying biological specimens for scanning electron microscopy processes. In general, CPD, especially from CO₂, was found to maintain high surface area of porous materials, such as silica aerogel, compared to regular drying.

In addition, recent advances in the technology explored the synthesis of porous materials by developing reactive processes in supercritical CO₂ fluid for synthesizing empty-pore three-dimensional metal-organic frameworks (MOFs), as well as processing disordered porous materials, such as polymers, aerogels, and concrete.

Supercritical fluid technology is also used successfully for protein micronization, encapsulation in particles, making biopolymeric microporous foam (using supercritical foaming), and supercritical impregnation for developing biomedical and pharmaceutical formulations or devices. For drying protein with polymers, SCF can be used in variety of ways to produce the biopolymer. The choice of the method is highly dependent on: interaction of the supercritical fluid (e.g., CO₂) with the active ingredient (e.g., protein), the solvent, and the coating material of interest or the polymer, where the interaction of the polymer with the supercritical fluid (e.g., CO₂) plays an important role.

Other drying methods can only preserve membrane properties for a limited storage time in the dried state. There exists a need for methods that produce dried composite materials that show consistent properties with storage, post-CPD, in the dried state.

### SUMMARY

Disclosed are methods for critical point drying composite materials that may be used for a variety of applications, including biomolecule separation and purification. Specifically, the CPD technique was used to maintain or nearly maintain the wet pore structure, surface area, and the permeability of the membrane in the dried state. After exposing the composite material to a supercritical fluid, the composite material dries as the supercritical fluid evaporates with reduced pressure.

In one aspect, the disclosure relates to methods of critical point drying a composite material, comprising the steps of:
a. providing a composite material comprising:
   i. a support member, comprising a plurality of pores extending through the support member; and
   ii. a macroporous cross-linked gel, wherein the macroporous cross-linked gel comprises a polymer formed from a reaction of one or more polymerizable monomers with one or more cross-linkers; the macroporous cross-linked gel is located in the pores of the support member; and said macropores of the macroporous cross-linked gel are smaller than the pores of the support member;
   wherein the composite material is wetted with an aqueous solution;
b. exchanging the aqueous solution with a polar organic solvent, such that the composite material is wetted with the polar organic solvent;
c. contacting the composite material with a supercritical fluid at a temperature and a pressure above the critical point of the supercritical fluid, wherein the gaseous and liquid phases are indistinguishable; and the polar organic solvent is miscible with the supercritical fluid; and
d. reducing the pressure;
thereby critical point drying the composite material.

In another aspect, the disclosure relates to methods of critical point drying a composite material, comprising the steps of:
a. providing a composite material comprising:
   i. a support member, comprising a plurality of pores extending through the support member; and
   ii. a macroporous cross-linked gel, wherein the macroporous cross-linked gel comprises a polymer formed from a reaction of one or more polymerizable monomers with one or more cross-linkers; the macroporous cross-linked gel comprises a plurality of ligands that are biological molecules or biological ions; the macroporous cross-linked gel is located in the pores of the support member; and said macropores of the macroporous cross-linked gel are smaller than the pores of the support member;
   wherein the composite material is wetted with an aqueous solution;
b. exchanging the aqueous solution with a polar organic solvent, such that the composite material is wetted with the polar organic solvent;
c. contacting the composite material with a supercritical fluid at a temperature and a pressure above the critical point of the supercritical fluid, wherein the gaseous and liquid phases are indistinguishable; and the polar organic solvent is miscible with the supercritical fluid; and
d. reducing the pressure;
thereby critical point drying the composite material.

### BRIEF DESCRIPTION OF THE FIGURES

**Fig. 1A** shows an exemplary composite material with layers of interleaf (e.g., a screen) in a spiral wound or roll assembly.
**Fig. 1B** shows an exemplary composite material (e.g., membrane discs) with layers of interleaf (e.g., a screen) in a membrane stack assembly in a CPD Holder.
**Fig. 2** shows Table 3 with exchange protocols with alcohol, buffer, and salt components.
**Fig. 3A** shows critical point drying (CPD) effect (circles) on membrane flux in comparison to other drying methods (without stabilizer, diamonds; and with glycerin, triangles).
**Fig. 3B** shows critical point drying effect (circles) on binding capacity in comparison to other drying methods (without stabilizer, diamonds; and with glycerin, triangles).
**Fig. 4A** shows membrane properties post-critical point drying storage with a membrane-to-ethanol ratio of 3.3.
**Fig. 4B** shows membrane properties post-critical point drying storage with a membrane-to-ethanol ratio of 4.3.
**Fig. 4C** shows membrane properties post-critical point drying storage with a membrane-to-ethanol ratio of 4.3.
**Fig. 4D** shows membrane properties post-critical point drying storage with a membrane-to-ethanol ratio of 5.6.
**Fig. 5A** shows the effect of extraction protocols on epoxy membrane IgG dynamic binding capacity post-CPD.
**Fig. 5B** shows the effect of extraction protocols on epoxy membrane flux post-CPD.
**Fig. 5C** shows the effect of extraction protocols on epoxy membrane IgG dynamic binding capacity post-CPD.
**Fig. 5D** shows the effect of extraction protocols on epoxy membrane flux post-CPD.
**Fig. 6** shows Table 4 with critical point drying parameters and related membrane ethanol extraction volume.
**Fig. 7** shows Table 5 with critical point drying parameters for membranes with varying binding capacities and permeabilities.
**Fig. 8A** shows the stability of various critical point dried epoxy membranes based on flux.
**Fig. 8B** shows the stability of various critical point dried Protein A membranes based on flux.
**Fig. 8C** shows the stability of various critical point dried epoxy membranes based on IgG dynamic binding capacity.
**Fig. 9A** shows the change of epoxy membrane pore size distribution in an exchanged wet sample.
**Fig. 9B** shows the change of epoxy membrane pore size distribution in a CPD sample after 25 days.
**Fig. 9C** shows the change of epoxy membrane pore size distribution in a sample dried at 50 °C for 10 min from EtOH after 1 day.
**Fig. 9D** shows the change of epoxy membrane pore size distribution in a sample dried at 50 °C for 10 min from water after 1 day.
**Fig. 10A** shows CPD effect on membrane flux and IgG dynamic binding capacity in comparison to solvent exchange and other drying methods (i.e., from water and from ethanol).
**Fig. 10B** shows CPD effect on epoxy membrane pore size properties in comparison to solvent exchange and other drying methods (from water and from ethanol).
**Fig. 11** shows surface area measurements of CPD epoxy membranes and an oven-dried membrane.
**Fig. 12** shows Table 6 with CPD run key parameters and related membrane alcohol extraction volume.
**Fig. 13A** shows CPD effect on membrane IgG dynamic binding capacity in comparison to oven drying method.
**Fig. 13B** shows CPD effect on membrane flux in comparison to oven drying method.
**Fig. 14A** shows the effect of exchange protocols with buffers on Protein A membrane IgG dynamic binding capacity with CPD.
**Fig. 14B** shows the effect of exchange protocols with buffers on Protein A membrane flux capacity with CPD.
**Fig. 15** shows the long-term stability of critical point dried Protein A membranes exchanged with an exemplary buffer protocol.
**Fig. 16A** shows the effect of exchange protocols with salts on Protein A membrane IgG dynamic binding capacity with CPD.
**Fig. 16B** shows the effect of exchange protocols with salts on Protein A membrane flux capacity with CPD.
**Fig. 17** shows the long-term stability of critical point dried Protein A membranes exchanged with an exemplary salt protocol.
**Fig. 18** shows a comparison of the effects of different exchange protocols on CPD Protein A membrane performance.
**Fig. 19A** shows binding capacity data for CPD membrane from AwB3 exchange protocol.
**Fig. 19B** shows flux data for CPD membrane from AwB3 exchange protocol.
**Fig. 20A** shows binding capacity data for CPD membrane from limited alcohol exchange protocol AwB3-SV.
**Fig. 20B** shows flux data for CPD membrane from limited alcohol exchange protocol AwB3-SV.
**Fig. 21A** shows binding capacity aging testing for CPD protein A membrane exchanged using protocols TP1-AWOB1 and TP2-AWOB1 (with 1 day storage in 20% alcohol).
**Fig. 21B** shows flux aging testing for CPD protein A membrane exchanged using protocols TP1-AWOB1 and TP2-AWOB1 (with 1 day storage in 20% alcohol).
**Fig. 22A** shows binding capacity aging testing for CPD protein A membrane exchanged using protocols TP1-AWOB1 and TP2-AWOB1 (with 7 day storage in 20% alcohol).
**Fig. 22B** shows flux aging testing for CPD protein A membrane exchanged using protocols TP1-AWOB1 and TP2-AWOB1 (with 7 day storage in 20% alcohol).
**Fig. 23A** shows binding capacity aging testing for CPD protein A membrane exchanged using protocols TP3-AWOB1 and TP4-AWOB1 (with 7 day storage in 16% alcohol in 20 mM phosphate, pH 7.5).
**Fig. 23B** shows flux aging testing for CPD protein A membrane exchanged using protocols TP3-AWOB1 and TP4-AWOB1 (with 7 day storage in 16% alcohol in 20 mM phosphate, pH 7.5).

### DETAILED DESCRIPTION

### Overview

Disclosed are methods for critical point drying composite materials that may be used for a variety of applications, including biomolecule separation and purification. In some embodiments, the composite material is for bio-affinity chromatography. In some embodiments, the composite material is a membrane. In some embodiments, the composite material is a porous polymeric gel membrane. Specifically, the CPD technique was used to maintain or nearly maintain the wet pore structure, surface area, and the permeability of the membrane in the dried state. Moreover, the critical point drying method produces dried membranes that show consistent properties with storage, post-CPD, in the dried state. In some embodiments, CPD is used as a drying approach to dry a bio-affinity media that has bioligand attached to porous gel or porous gel composite materials that can be used, among other applications, as chromatographic media for bio-separation. Specifically, the CPD technique is used to dry the conjugated protein-polymer material while maintaining the structure and the functionality of the bio-affinity media. In contrast, other drying methods can only preserve membrane properties for a limited storage time in the dried state.

### Definitions

For convenience, before further description of the present invention, certain terms employed in the specification, examples and appended claims are collected here. These definitions should be read in light of the remainder of the disclosure and understood as by a person of skill in the art. Unless defined otherwise, all technical and scientific terms used herein have the same meaning as commonly understood by a person of ordinary skill in the art.

In describing the present invention, a variety of terms are used in the description. Standard terminology is widely used in filtration, fluid delivery, and general fluid processing art.

The articles "a" and "an" are used herein to refer to one or to more than one (i.e., to at least one) of the grammatical object of the article. By way of example, "an element" means one element or more than one element.

The terms "comprise" and "comprising" are used in the inclusive, open sense, meaning that additional elements may be included.

The term "including" is used to mean "including but not limited to." "Including" and "including but not limited to" are used interchangeably.

The term "polymer" is used to mean a large molecule formed by the union of repeating units (monomers). The term polymer also encompasses copolymers.

The term "co-polymer" is used to mean a polymer of at least two or more different monomers. A co-polymer can be comprised of a cross-linker and a monomer, if the cross-linker is a difunctional monomer.

The term "supercritical fluid" (SCF) is used to mean any chemical substance that is placed at a temperature and pressure above its critical point, where the gaseous and liquid phases are not distinguishable. An important feature of supercritical fluids is that in the critical region the fluids show density like liquids, while the viscosity and diffusivity are similar to gases. Therefore, supercritical fluids have good mixing and mass transfer properties, and their capacity to solubilize any solute is not-surprisingly sensitive to both temperature and pressure. This variation of solubility at different temperature and pressure provide the opportunity to use supercritical fluids in several applications where solubilizing and precipitation/separation of solute can be controlled by changing the critical region conditions.

The term "Protein A" or "PrA" is used to mean a bacterial protein from *Staphylococcus aureus*, a Protein A derivative, or a recombinant Protein A with the capacity to bind mammalian antibodies of class immunoglobulin G (IgG) with high affinity. The gene for Protein A has been cloned and expressed in *Escherichia coli* allowing for the production of large quantities of recombinant Protein A and Protein A derivatives. In some embodiments, Protein A is an affinity ligand. In some embodiments, Protein A is a protein, peptide, or recombinant protein comprising a ligand that binds monoclonal antibodies (e.g., IgG antibodies) and a moiety that can form a covalent bond with a pendant reactive functional group (e.g., thiol of Cys, or amine of Lys). In some embodiments. Protein A is a protein, peptide, or recombinant protein comprising a ligand that binds with the Fc domain of an antibody and a moiety that can form a covalent bond with a pendant reactive functional group. In some embodiments. Protein A is a protein, peptide, or recombinant protein comprising a ligand that binds with the Fab domain of an antibody and a moiety that can form a covalent bond with a pendant reactive functional group.

### Methods of Use

Composite materials comprising pendant reactive functional groups can be grafted with ligands through chemical reactions. For example, porous membranes containing active epoxy pendant groups can be grafted with bio-affinity ligands through nucleophilic conjugation reactions with suitable groups present on bio-affinity ligands. The conjugated membranes can serve as chromatographic media useful for bio-affinity separations of specific targets, such as monoclonal antibodies. To have highly productive separation performance, the membrane platform should maintain a porous structure that produces sufficient permeability to flow and desired target binding capacity, and these features should be stable with storage.

In general, bio-affinity chromatographic media depends on the immobilized ligand specificity that allows it to bind selectively a specific target among other analytes, which can be released in subsequent steps, using mobile phases that reduce the binding forces between the ligand and the target. This bioactivity of the ligand depends largely on the geometrical structure as well as chemical structure of the active center, and therefore any changes in either the chemical structure or stereo structure are likely to impact the binding capacity of the chromatographic media.

One of the major challenges in making chromatographic media is to immobilize specific ligands at the interface and maintain their bio-activity, especially in dry form. In general, drying protein molecules exposes the biomolecule to various physical forces due to water removal and intra-molecular interaction forces that put pressure on the three-dimensional structure of the large molecule, which likely to result in changes in the 3D structure that are irreversible.

Many techniques were examined for protein drying, including freeze-drying and the emerging SCF drying technique. Historically, freeze-drying (also known as lyophilization) is the preferred method to dehydrate proteins. However, there are stresses associated with the freezing process itself, especially in the freezing step, which can pose risk for protein denaturation. Supercritical fluid drying is relatively a new technique that also comes with different stresses on the protein structure as the high pressure associated with SCF process might influence the protein stability, and the use of organic solvents may result in denaturation or aggregation.

For immobilized ligands or biomolecules, the task is more complicated as additional forces due to various interactions with the media surface (hydrophobic and ionic interactions) amplify the drying effects and likely reduces the ligand bioactivity. It is essential to control the drying process in order to maintain bioactivity, especially if the media surface is also affected by the drying process. While freeze-drying is effective in drying protein, an immobilized biomolecule may require a drying technique that can dry both the immobilized molecule and the platform as well, especially if the latter is a material sensitive to the drying technique.

The membrane platform is usually made by in-situ polymerization of acrylate/acrylamide monomers and crosslinker on within non-woven fibrous polymer substrate to obtain porous enforced-gel material. For example, epoxy-containing membrane was made using acrylate and acrylamide monomers and crosslinkers on non-woven fibrous polymer substrate to obtain a porous gel material. The porosity and the structure of the porous gel of the membrane was tuned by a thermal treatment process to produce desired properties, such as permeability and protein binding capacity.

In some embodiments, porous epoxy membrane has active epoxy groups and can be grafted with bio-affinity ligands through nucleophilic conjugation reaction that utilize amino groups on the bio-molecule. In some embodiments, the epoxy membrane was conjugated with a bioligand through a two-step process of coupling the biomolecule through a nucleophilic addition reaction, followed by a capping step to reduce active epoxy groups on the surface. In some embodiments, the conjugated membrane can serve as a chromatographic platform to perform bio-affinity separation of specific targets, such as monoclonal antibodies. In order to have good separation performance, the conjugated membrane should have good porous structure with high surface area and good permeability, as well as maintaining immobilized ligand bioactivity.

Drying the conjugated membrane can increase the stability of the ligand, increase the shelf life of the biopolymer, facilitate the handling of the material, and reduce the risk of contamination. Two components must be considered while drying the bioaffinity media: the stability of the immobilized ligand and the stability of the polymer carrier.

However, the properties of such obtained structure in the dry state was found to change over time. Without being bound by theory, the sensitivity of the membrane gel to the drying process may be related to the pore structure of gel, which may be irreversibly altered during drying processes. In particular, drying the membrane gel from an aqueous wetting phase, which has a relatively high surface tension, may expose the gel structure to high shear forces during the removal of the water in the drying step.

For example, the drying of the conjugated bioaffinity membrane is found to be challenging as the functionality and chromatographic performance of the membrane changes with drying process and continues to deteriorate with time. This has been observed for epoxy group containing membranes which have shown limited stability when dried either at room temperature or at higher temperatures (40-50 °C). In addition to the membrane platform sensitivity, ligand susceptibility to drying process stresses cannot be ignored and can be responsible for the loss of bioactivity.

Without being bound by theory, it is also expected that the ligand may be impacted by water removal, which expose the protein to various intra- and inter-molecular interaction forces, in the vicinity of the membrane surface. Biomolecule-surface interaction forces can alter the spatial structure of the protein, leading to denaturation and loss of activity.

Critical point drying provides the opportunity to dry the membrane from a supercritical fluid (e.g., carbon dioxide) phase under very low surface tension condition that minimizes these forces on the membrane gel structure and thereby maintains the wet properties. That is, SCF provides an essentially zero surface tension environment to dry material due to the unique fluid properties at critical conditions, where gaseous and liquid phases can be indistinguishable.

Carbon dioxide is a good supercitical fluid candidate for critical point drying as it can reach a supercritical state under relatively mild conditions, compared to other liquid or gases. For example, the critical point of water is at 374 °C and 228 bar, and these conditions may result in irreversible changes to many materials of interest. That is, the critical point of water presents a more difficult and extreme set of conditions to reach that may affect the polymer and the ligand, not only physically but possibly chemically. In contrast, the critical point of carbon dioxide is at 31.2 °C and 73.8 bar, which are much less likely to irreversibly alter sample properties and require less stringent equipment design and controls. In addition, the CO₂ is non-toxic and cost-efficient for large scale processes.

However, due to its immiscibility with water, it is not possible to transition from an aqueous phase directly to a CO₂ drying phase. Therefore, the water in the composite material to be dried (e.g., the membrane) must be exchanged with a polar organic solvent (such as an alcohol or a ketone) first to displace the water with the organic solvent. The exchange fluid therefore must have good miscibility with water to permit the exchange process, and be miscible with CO₂ to allow a final exchange of the membrane into the CO₂ drying fluid. In some embodiments, one hurdle to overcome is the expected negative effect of exposing biomolecules to these organic solvents.

In the final step, the temperature of the chamber where the material of interest (membrane) and the CO₂ drying fluid are enclosed, is increased beyond the critical point temperature and pressure to the supercritical region, after which controlled evaporation by reducing pressure takes place, and drying occurs in the absence of surface tension forces.

It is known that some proteins can be stable in some organic solvents, provided that the water content in the solvent is low (usually less than 10-20%). It is also known that minimizing conformational changes during a drying process can help with stabilizing a protein during a drying process. Therefore, in some embodiments, to have a successful CSF drying process, the exchange protocol must include an initial step of buffer/salt exchange to provide enough ions (to reduce intra-molecule ionic interactions during CSF drying), and to strictly use gradients of organic solvent that have low water content (<10%).

In one aspect, the disclosure relates to methods of critical point drying a composite material, comprising the steps of:
a. providing a composite material comprising:
   i. a support member, comprising a plurality of pores extending through the support member; and
   ii. a macroporous cross-linked gel, wherein the macroporous cross-linked gel comprises a polymer formed from a reaction of one or more polymerizable monomers with one or more cross-linkers; the macroporous cross-linked gel is located in the pores of the support member; and said macropores of the macroporous cross-linked gel are smaller than the pores of the support member;
   wherein the composite material is wetted with an aqueous solution;
b. exchanging the aqueous solution with a polar organic solvent, such that the composite material is wetted with the polar organic solvent;
c. contacting the composite material with a supercritical fluid at a temperature and a pressure above the critical point of the supercritical fluid, wherein the gaseous and liquid phases are indistinguishable; and the polar organic solvent is miscible with the supercritical fluid; and
d. reducing the pressure;
thereby critical point drying the composite material.

In another aspect, the disclosure relates to methods of critical point drying a composite material, comprising the steps of:
a. providing a composite material comprising:
   i. a support member, comprising a plurality of pores extending through the support member; and
   ii. a macroporous cross-linked gel, wherein the macroporous cross-linked gel comprises a polymer formed from a reaction of one or more polymerizable monomers with one or more cross-linkers; the macroporous cross-linked gel comprises a plurality of ligands that are biological molecules or biological ions; the macroporous cross-linked gel is located in the pores of the support member; and said macropores of the macroporous cross-linked gel are smaller than the pores of the support member;
   wherein the composite material is wetted with an aqueous solution;
b. exchanging the aqueous solution with a polar organic solvent, such that the composite material is wetted with the polar organic solvent;
c. contacting the composite material with a supercritical fluid at a temperature and a pressure above the critical point of the supercritical fluid, wherein the gaseous and liquid phases are indistinguishable; and the polar organic solvent is miscible with the supercritical fluid; and
d. reducing the pressure;
thereby critical point drying the composite material.

In some embodiments, the supercritical fluid is selected from the group consisting of carbon dioxide, water, methane, ethane, propane, ethylene, propylene, methanol, ethanol, acetone, and nitrous oxide. In some embodiments, the supercritical fluid is not water. In some embodiments, the supercritical fluid is selected from the group consisting of carbon dioxide, methane, ethane, propane, ethylene, propylene, methanol, ethanol, acetone, and nitrous oxide. In some embodiments, the supercritical fluid is carbon dioxide.

In some embodiments, one or more of the following parameters selected from the group consisting of total amount of supercritical fluid relative to sample volume, number of purge cycles, temperature during contact with the supercritical fluid, the exchange rate with the supercritical fluid, the temperature during the evaporation step, the heating rate to increase temperature, and the rate of the pressure reduction can be varied in the methods disclosed herein. For example, parameters that can be varied to affect the drying process include: 1) the total amount of liquid CO₂ (or number of purge cycles) relative to sample volume, 2) the chamber temperature during CO₂ exchange, the liquid CO₂ exchange rate, 3) the temperature set for the drying step (typically higher than Tc), 4) the heating rate at which the chamber temperature increases, and 5) the CO₂ pressure decline rate during the evaporation step.

In some embodiments, the polar organic solvent is selected from the group consisting of acetone, acetonitrile, ammonia, t-butanol, n-propanol, ethanol, methanol, and acetic acid.

In some embodiments, the support member comprises a polymeric material selected from the group consisting of polysulfones, polyethersulfones, polyphenyleneoxides, polycarbonates, polyesters, cellulose, and cellulose derivatives.

In some embodiments, the composite material is a membrane.

In some embodiments, the composite material is in contact with one or more interleaf layers. In some embodiments, the one or more interleaf layers are selected from the group consisting of screen, polypropylene, polyethylene, and paper.

In some embodiments, the cross-linked gel is a neutral hydrogel, a charged hydrogel, a polyelectrolyte gel, a hydrophobic gel, a neutral gel, or a gel comprising functional groups.

In certain embodiments, the composite materials used as membranes in the present invention are described in US Patent Nos. 7,316,919; 8,206,958; 8,187,880; 8,211,682; 8,652,849; 8,192,971; 8,206,982; 8,367,809; 8,383,782; 8,133,840; 9,962,691; and 10,357,766; and US Patent Application Serial Nos. 14/190,650, 16/055,786, and 16/516,5 00.

In some embodiments, the macroporous cross-linked gel comprises macropores having an average size of about 10 nm to about 3000 nm.

In some embodiments, the pores of the support member have an average pore diameter of about 0.1 µm to about 50 µm.

In some embodiments, the cross-linked gel comprises pendant reactive functional groups. In some embodiments, the pendant reactive functional groups are selected from the group consisting of aldehydes, amines, carbon-carbon double bonds, carbon-carbon triple bonds, epoxides, hydroxyls, thiols, anhydrides, azides, reactive halogens, acid chlorides, and a mixture thereof. In some embodiments, the pendant reactive functional groups are epoxides. In some embodiments, the one or more monomers comprising a pendant reactive functional group are selected from the group consisting of glycidyl methacrylate, acrylamidoxime, acrylic anhydride, azelaic anhydride, maleic anhydride, hydrazide, acryloyl chloride, 2-bromoethyl methacrylate, and vinyl methyl ketone.

In some embodiments, the cross-linked gel comprises a plurality of ligands. In some embodiments, the cross-linked gel comprises a plurality of ligands comprising a functionality. In some embodiments, the functionality is selected from the group consisting of cationic, anionic, hydrophobic, hydrophilic, thiophilic, hydrogen bond donating, hydrogen bond accepting, pi-pi bond donating, pi-pi bond accepting, metal chelating, a biological molecule, and a biological ion.

In some embodiments, the plurality of ligands are biological molecules or biological ions selected from the group consisting of albumins, lysozyme, viruses, cells, γ-globulins of human and animal origins, immunoglobulins of both human and animal origins, proteins of recombinant or natural origin including, polypeptides of synthetic or natural origin, interleukin-2 and its receptor, enzymes, monoclonal antibodies, antigens, lectins, bacterial immunoglobulin-binding proteins, trypsin and its inhibitor, cytochrome C, myoglobulin, recombinant human interleukin, recombinant fusion protein, Protein A, Protein G, Protein L, Peptide H, nucleic acid derived products, DNA of either synthetic or natural origin, and RNA of either synthetic or natural origin. In some embodiments, the plurality of ligands are biological molecules or biological ions selected from the group consisting of γ-globulins of human and animal origins, immunoglobulins of both human and animal origins, proteins of recombinant or natural origin including, polypeptides of synthetic or natural origin, monoclonal antibodies, bacterial immunoglobulin-binding proteins, recombinant fusion protein, Protein A, Protein G, and Protein L. In some embodiments, the plurality of ligands are biological molecules or biological ions selected from the group consisting of a polypeptide, a protein, a recombinant protein, a bacterial immunoglobulin-binding protein, a recombinant fusion protein, Protein A, Protein G, Protein L, and Peptide H. In some embodiments, the plurality of ligands are Protein A.

In some embodiments, the composite materials (e.g., membranes) comprise polymers that contain various ligands useful for affinity chromatography. For example, chromatographic separation of biomolecules (e.g., proteins). For example, this approach can be used to introduce to the composite materials (e.g., membrane) ion exchange functionalities (e.g., carboxylate, sulfonate, quaternary ammonium, amine), hydrophobic interaction moieties (such as octyl group by using 1-octanethiol or 1-octene), and biomolecules for bio-affinity chromatography (such as cysteine-protein A for monoclonal antibody purification).

In some embodiments, the composite materials demonstrate instability in and after conventional drying processes. In some embodiments, critical point drying allows removal of water without damaging the composite material. In some embodiments, critical point drying allows removal of water without damaging the membrane comprising ligands. In some embodiments, the invention relates to methods of critical point drying of composite materials such that the composite materials show equivalent properties to initial water wet composite materials.

In some embodiments, the methods of critical point drying of composite materials improve composite material storage, assembly of a membrane into the final device, and stable shipping with reduced bioburden load.

### EXEMPLIFICATION

The following examples are provided as illustrations. It will be understood, however, that the specific details given in each example have been selected for purpose of illustration and are not to be construed as limiting the scope of the disclosure. Generally, the experiments were conducted under similar conditions unless noted.

### Example 1 - General materials and methods

### Chemicals

Liquified carbon dioxide (medical grade 99.99%) was obtained from PraxAir Canada Inc., anhydrous alcohol reagent (<0.005% water) and calcium chloride were obtained from MilliporeSigma, and buffer solution 10X PBS liquid concentrate (Grow Cells) was purchased from VWR.

### Protein

Human IgG, affinity purified, was obtained from Innovative Research Inc. (Novi, MI, USA).

### Membrane Material

Epoxy membranes were made by polymerizing acrylate and or acrylamide monomers and crosslinkers within a support mesh material in a UV initiated reaction. Various functional membranes containing protein binding groups (e.g. ion exchange, hydrophobic interaction and hydrophilic interaction) may be produced in a single polymerization step by introducing suitable functional polymerizable groups into the gel polymerization solution. The wet washed membranes may also be subjected to an additional thermal treatment step to tune their performance, final pore structure, and permeability.

Subsequently, the epoxy membrane was conjugated with Protein A ligand through nucleophilic addition reaction where mostly a nucleophilic group on the ligand molecule (amino or thiol groups) open the oxirane ring and covalently bind the bioligand to the membrane gel.

Other ligands can also be conjugated to target other bio-molecules or entities such as viruses.

In a typical conjugation protocol, a membrane disc (25 mm) was mounted in a Natrix stainless steel holder then connected to an AKTA chromatography instrument, where the membrane was equilibrated with PBS, then the ligand (Protein A) solution in phosphate buffer solution was passed through the membrane under selected flowrate(s) for an appropriate length of time. This was followed by buffer wash step then the unreacted epoxy groups of the membrane were passivated by flowing a quenching solution through the membrane, followed by a buffer wash step remove residual quenching solution.

### Membrane Performance Testing

Membranes were evaluated for their permeability (at both pre and post-conjugated states when ligands were conjugated to membranes containing pendant reactive groups) and for their binding capacity for specific biomolecule target.

For permeability, flux testing was carried out at a defined pressure (100 kPa), with water typically used as the test fluid, to estimate the amount of fluid passing through specific membrane surface area, and the final permeability results were expressed in kg/m²h.

Membrane binding capacity was measured by determining the amount of selected protein captured by a test volume membrane under defined flow conditions (i.e. dynamic binding capacity, dBC) and expressed as mg protein bound/mL of membrane.

For membrane bio-affinity, binding capacity was measured by determining the amount of IgG protein captured by the active membrane volume at 10% breakthrough absorbance the passing testing solution of the target molecule in buffer (IgG in this case). The dynamic binding capacity (dBC) expressed as mg of target molecule /mL of membrane volume.

### Membrane Format

### Format A

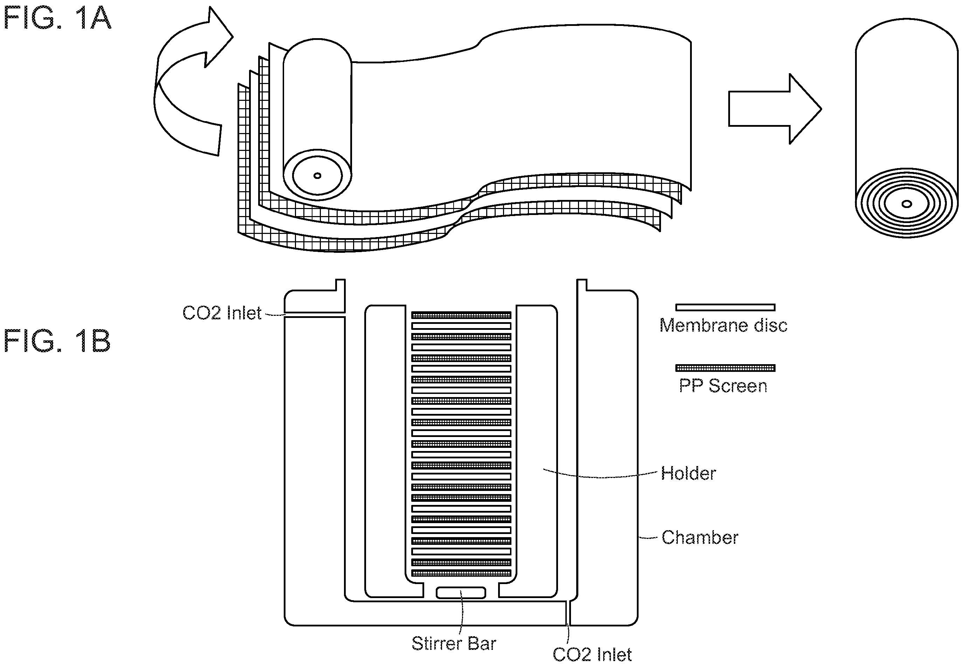

Membrane samples were generally solvent exchanged and critical point dried in a roll format. Membrane strips were layered with strips of polypropylene screen of similar width and slightly longer length (2-3 cm longer) then rolled up (Fig. 1A). The rolls were made in dimensions that fit into the critical point drying (CPD) chamber sample holder (mostly 4 cm in width and OD of ~ 4.5 cm) or in some cases fits directly in the instrument chamber itself (width <5.5cm and OD below 5.8cm).

### Format A'

Membrane samples were exchanged and dried in hand-made roll format. Protein A Membrane strips (~ 4x36 cm) were layered with PP screens of similar width and slightly longer strips (2-3 cm longer) then rolled up (Fig. 1A). The screens were used to improve the exchange process and process consistency. The rolls were made in dimensions that fit the instrument holder (mostly 4 cm in width and OD of ~ 4.5 cm) or in some cases fits directly in the instrument chamber itself (width <5.5cm and OD below 5.8 cm). A wire was used to keep the interleafed roll together during the exchange process, which was carried out in 200 mL beaker, then was removed just before placing the roll into the holder (or the chamber for the larger roll setup).

### Format B

Membrane samples were critical point dried as an interchanging stack of membrane and screen discs of the same diameter. After being fully exchanged in a separate container with the appropriate solution volume in each step according to the protocol, the membrane discs (25 mm) were stacked in a modified sample block (with an ID of 25 mm) with screen discs of same diameter interchangeably, as shown in Fig. 1B. The screens discs serve as separators to promote better mixing and accessibility during the solvent exchange step, as well as the CPD drying process in which exchanging alcohol with CO₂ is crucial for successful drying. The stack was soaked briefly in fresh amount of anhydrous alcohol before being mounted onto the exchange chamber of the CPD instrument.

### Water/Ethanol Exchange Protocol

Carbon dioxide critical pressure and temperature are 73.8 bar and 31.2°C, respectively. These mild conditions to reach for supercritical fluid status makes carbon dioxide a suitable choice for performing critical point drying while preserving fragile structures during the process. However, the poor miscibility of water in CO₂ makes it necessary to exchange the water with ethanol, a more CO₂ miscible intermediate liquid phase that can be easily replaced with liquid carbon dioxide prior to the drying process.

A membrane roll was usually transferred into the ethanol exchange fluid via a water ethanol gradient to ultimately replace the aqueous phase in the membrane with anhydrous alcohol that possess very low water content. Several protocols were examined that have different number of steps, variable alcohol content, and residence time(s). Mainly, two protocols were examined: Protocol A with five short steps with gradual decrease in water content (Table 1) and protocol B with eight steps (Table 2).

**Table 1. Solvent Exchange Protocol A**

| Step | Alcohol Solution % | Soaking Time (min) |
|---|---|---|
| 1 | 30 | 10 |
| 2 | 70 | 10 |
| 3 | 90 | 10 |
| 4 | 100 | 10 |
| 5 | 100 | 10 |

**Table 2. Solvent Exchange Protocol B**

| Step | Alcohol Solution % | Soaking Time (min) |
|---|---|---|
| 1 | 30 | 10 |
| 2 | 70 | 10 |
| 3 | 70 | 10 |
| 4 | 90 | 10 |
| 5 | 90 | 10 |
| 6 | 100 | 10 |
| 7 | 100 | 10 |
| 8 | 100 | 10 |

Despite CO₂ being the most suitable choice for CPD process, its poor miscibility with water makes it necessary to replace the aqueous phase (buffer in this case) with alcohol, a more miscible liquid phase that can be easily replaced with liquid carbon dioxide. Typically, the exchange protocol for CPD involves exchanging the subject in ascending gradient solution of alcohol in water, ending with anhydrous alcohol soak/exchange to remove all water before going to the SCF step and eventually evaporating the CO₂ by reducing the pressure to make the transition from supercritical phase into gaseous phase.

However, because the conjugated form has a ligand attached to the membrane, the exchange protocol was modified to minimize or mitigate the expected negative effects of exposure of the ligand molecule to organic solution(s). First, the initial step of the exchange protocol is designed to promote enriching the membrane and the ligand with counter ions by performing an extended soak in a buffer solution. Secondly, instead of transitioning the membrane gradually to the anhydrous alcohol phase, the membrane was transitioned from a 100% aqueous phase directly into 90% alcohol-content phase to avoid exposing the protein to alcohol mixtures of high-water content that may promote denaturation. Finally, the exchange hold times were limited to avoid prolonged exposure to alcohol solution(s).

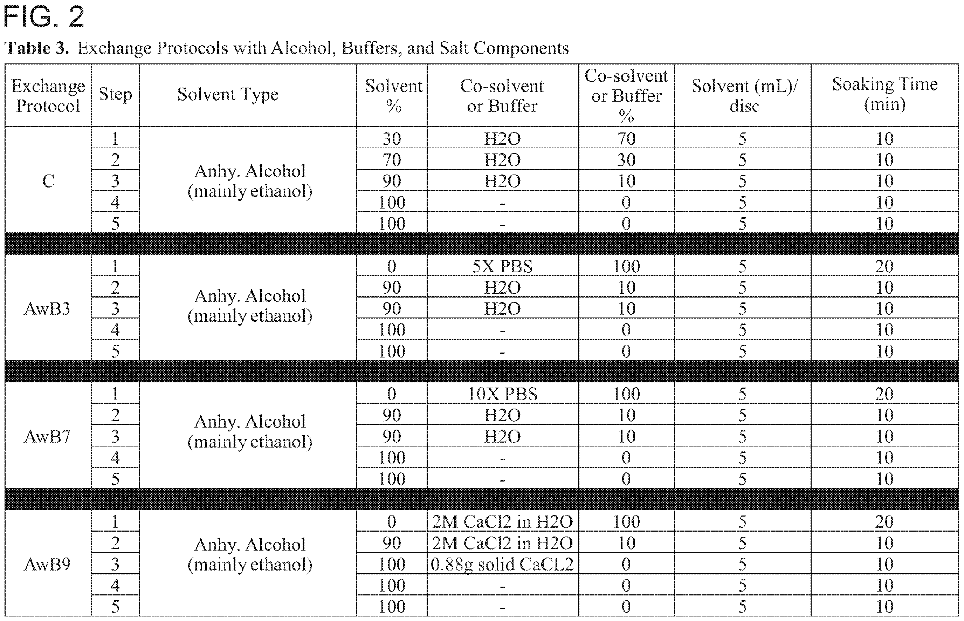

Several protocols were examined with different steps and variable alcohol solutions and will be detailed in the examples section. To demonstrate the importance of the modified-exchange protocol, a typical protocol that was used in CPD of epoxy membrane (Protocol C - Table 3; Fig. 2) was also tested on Protein A conjugated membrane as will be detailed in the following example

As described earlier, 25 mm discs were exchanged in a separate container with the appropriate solution/volume according to the specified exchange protocol. When the exchange steps were finished, the membrane coupons were stacked with screen separators and the whole holder was placed in a 200 mL beaker with fresh anhydrous alcohol solution, then finally transferred into the instrument chamber.

### Critical Point Drying

The critical point drying (CPD) process was carried out using a Leica EM CPD300 equipped with a liquid carbon dioxide tank that has siphon tube for liquid CO₂ discharge (~55 bar) and a separator bottle to collect extracted ethanol from the outlet CO₂/alcohol mixture. In a typical auto-mode run, the membrane roll was placed in the cylindrical holder within the chamber, and anhydrous ethanol solution was added until the sample is mostly covered with ethanol, then the lid was tightened to seal the chamber. Alternatively, the chamber could be cooled first to the set temperature, then the membrane roll was loaded up as previously described.

The instrument program started the process automatically by cooling down the chamber to the designated filling temperature (10 or 15 ⁰C), then feeding in the liquid CO₂ to fill the chamber. After a pre-defined delay period, which was set to allow initial mixing of alcohol with carbon dioxide liquid, the alcohol purging process starts by injecting liquid CO₂ over multiple cycles (12-99 cycles) where fresh CO₂ replaces a portion of the solution mixture in the chamber in each cycle and in a continuous manner, while maintaining the pressure in the chamber within a certain range (maintaining CO₂ in the liquid state). In addition to setting the number of exchange cycles, it is also possible to set the speed at which CO₂ is fed through the chamber.

When the purging process was complete, the instrument switched to a heating step where the chamber temperature was increased beyond the critical temperature (34-36 ⁰C) and the pressure was set above the critical pressure (> 74 bar) so the membrane was now immersed in supercritical CO₂ liquid. In the final step, the evaporation process took place by releasing the CO₂ slowly and reducing the pressure while maintaining the temperature, which allowed the transition from supercritical phase to the gaseous phase in the absence of any significant surface tension forces. The process ended by bringing the final pressure to 1 bar at which the dry membrane would be removed from the chamber. The instrument allows some control over the heating/evaporation step as the speed can be set at three different levels.

Several parameters can be varied to affect the drying process, including: 1) the total amount of liquid CO₂ (or number of purge cycles) relative to sample volume, 2) the chamber temperature during CO₂ exchange, the liquid CO₂ exchange rate, 3) the temperature set for the drying step (typically higher than Tc), 4) the heating rate at which the chamber temperature increases, and 5) the CO₂ pressure decline rate during the evaporation step. Without implying any limitations or restrictions on any of the CPD process parameters, only selected protocols were explored, solely for demonstrating the concept of using a CPD process to obtain a storage-stable dry membrane.

### Pore Size Measurements

Membrane pore size (diameter) was measured using 3G Porometer, a capillary flow porometer (Quantochrome Instruments, Boynton Beach, FL), operated by 3GWin software (V2.2). A small disc of dry membrane (1.8 cm diameter) was soaked in Porofil wetting liquid (Quantochrome Corp.), surface tension=16 dynes/cm) for 10 min, then it was gently squeezed between two pre-wetted filter paper discs (Whatman 5-70 mm) to remove excess solution, and the thickness of the wetted membrane was determined using a micrometer. The membrane disc was placed on stainless steel mesh support disc within the designated holder, with the membrane facing up. The metal cover was then gently placed on the holder and closed, then the test was carried out within the pressure range of between 0-200 psi.

### Example 2 - Comparing critical point drying of epoxy-containing membranes to other drying methods and their effect on performance during storage

Various batches of epoxy membranes that had similar permeabilities, and their bioligand derivatized forms having comparable protein binding capacities, were dried using a two-step critical point drying process. In the first step, the membranes in roll format were subjected to a water/ethanol exchange process, where the membrane roll was placed in the exchange solvent of certain alcohol content for specified time then the reagent was decanted and fresh specified amount of the alcohol solution of the following step was added, as outlined in the exchange protocol B (Table 2). In the second step, the ethanol-wet membrane roll was placed in the critical point drying instrument chamber (Leica CPD300) and subjected to a purging process to replace ethanol with liquid CO₂, followed by transferring the drying chamber conditions to the supercritical state (34 °C and 79 bar), and finally releasing the pressure to evaporate the supercritical fluid CO₂.

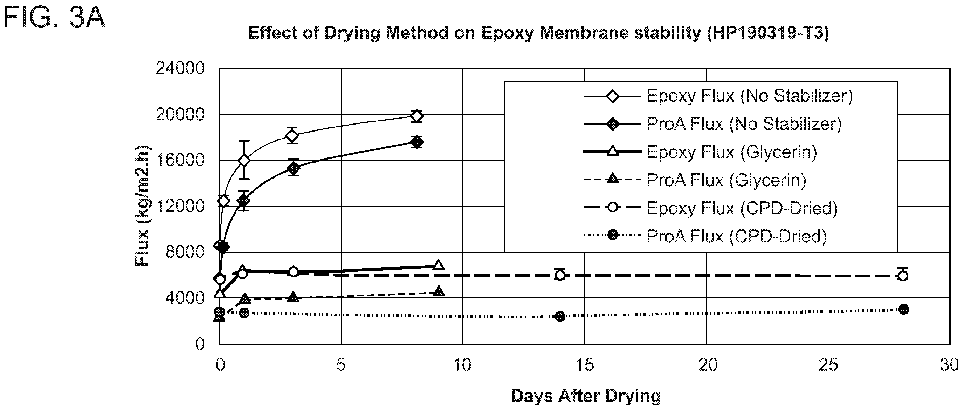

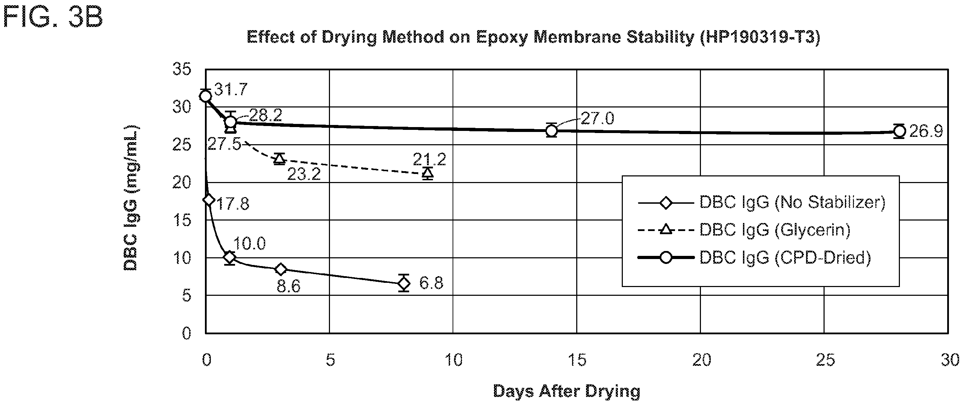

To illustrate the impact of the CPD process on stability compared to other methods, an epoxy-containing membrane was dried in an oven (50 °C - 15 min) and in another experiment it was soaked in 50% glycerin solution for 30 min then dried in the oven (50 °C - 15 min). The results (Fig. 3A and Fig. 3B) demonstrate the effectiveness of CPD method to preserve the wet membrane performance, in comparison to oven drying methods (with or without glycerin stabilizer). There was an initial small drop in the binding capacity (5-10%) of the critical point dried membrane, compared to wet membrane (Fig. 3B). However, membrane characteristics were essentially unchanged for the following storage-testing period.

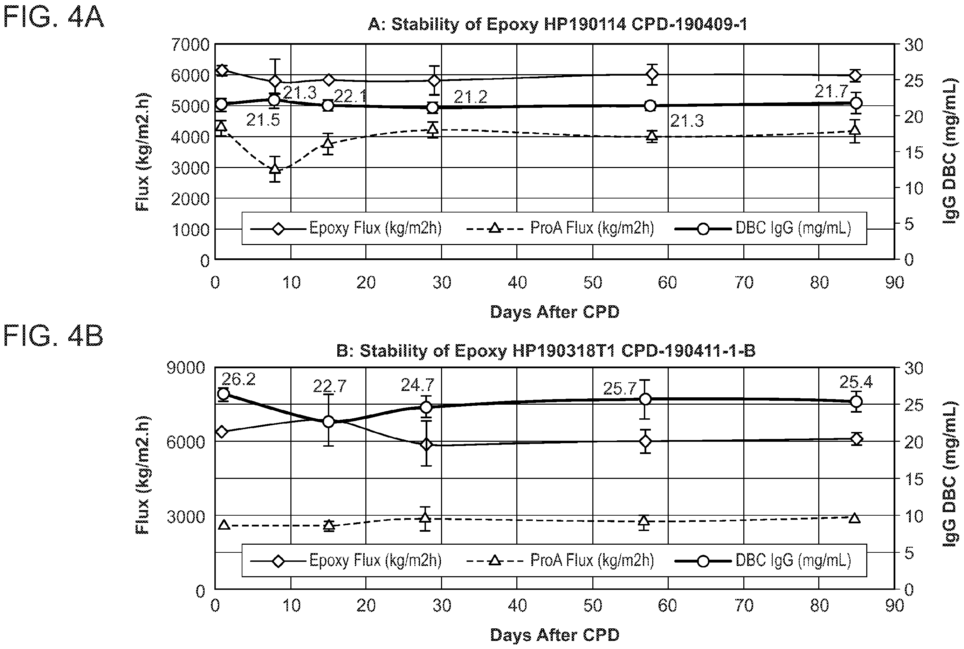

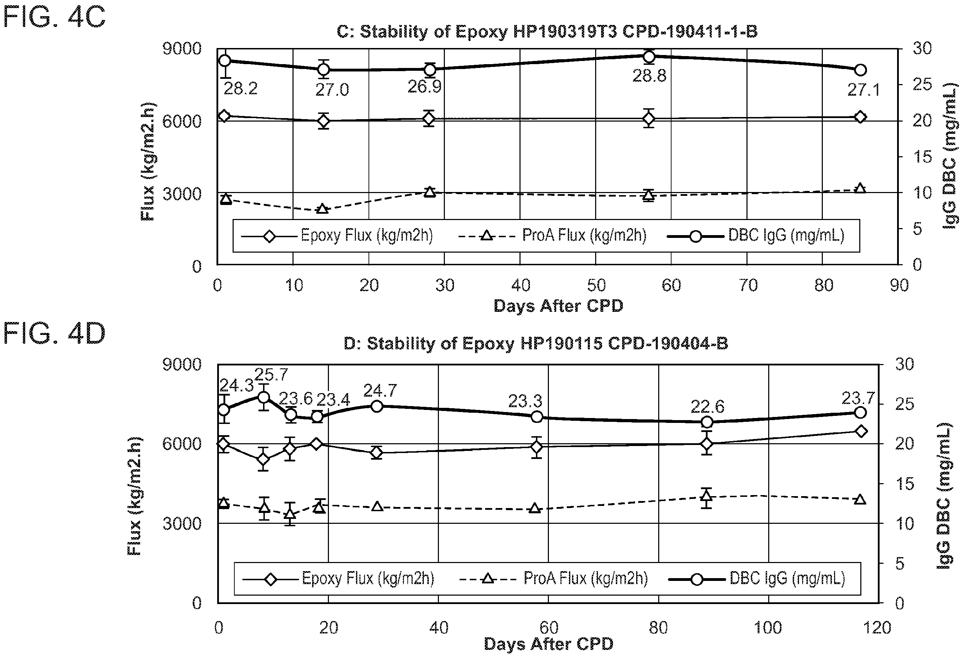

Long-term storage testing, as shown in Fig. 4A - 4D, demonstrated that critical point dried membrane (epoxy form) permeability (flux) did not significantly change over the 90 day test period examined. Moreover, the critical point dried membranes were proven to produce derivatized protein A membrane with similar performance (flux and binding capacity) over the same 90 day storage period. This is strikingly different from the behavior of the oven-dried membrane over even a short storage time.

### Example 3 - Critical point drying of epoxy-containing membranes using different exchange protocols

To have a successful drying process using supercritical fluid, water has to be removed and replaced with ethanol. There are several factors expected to affect the water removal process: water content in the ethanol exchange solution, exchange time, number of exchange steps, the interleaf screen material, and finally the membrane-to-solution ratio.

Two different exchange protocols, with gradient ethanol content, were examined. In Protocol A, the membrane is exchanged with ethanol solutions to replace water in membrane with alcohol through a five-step exchange protocol that uses solutions of gradually decreasing of water content to eventually reach exchanging steps with anhydrous ethanol reagent. In protocol B, the exchange process takes place over eight exchange steps thoroughly to replace water in the membrane with ethanol. The two protocols A and B are outlined in Table 1 and Table 2, respectively.

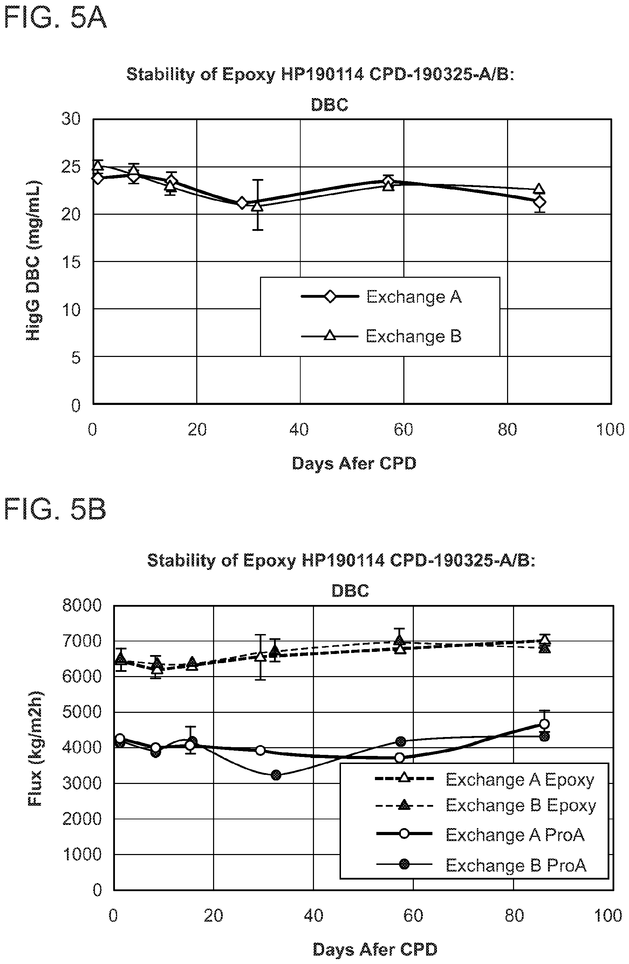

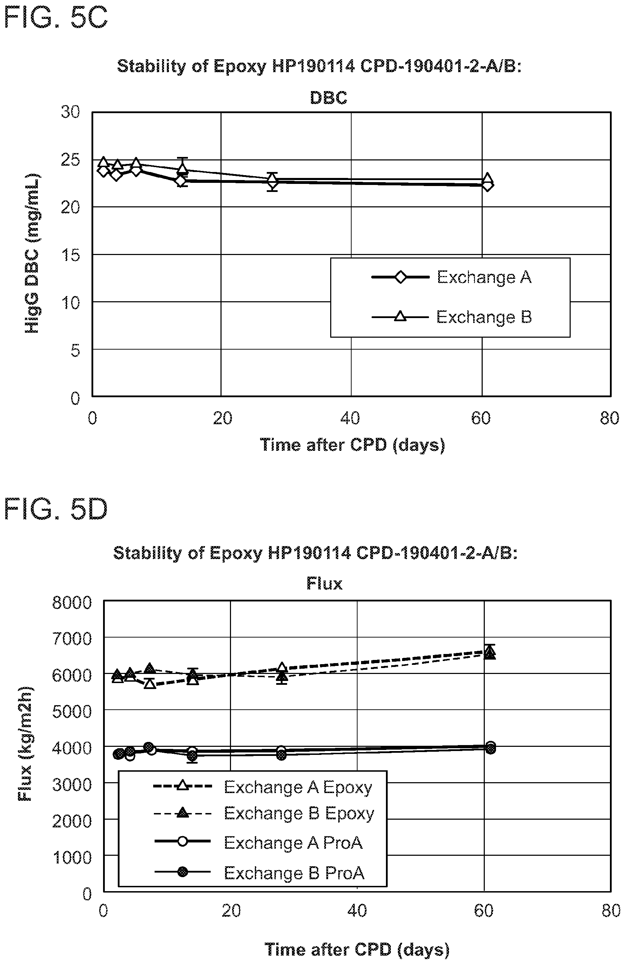

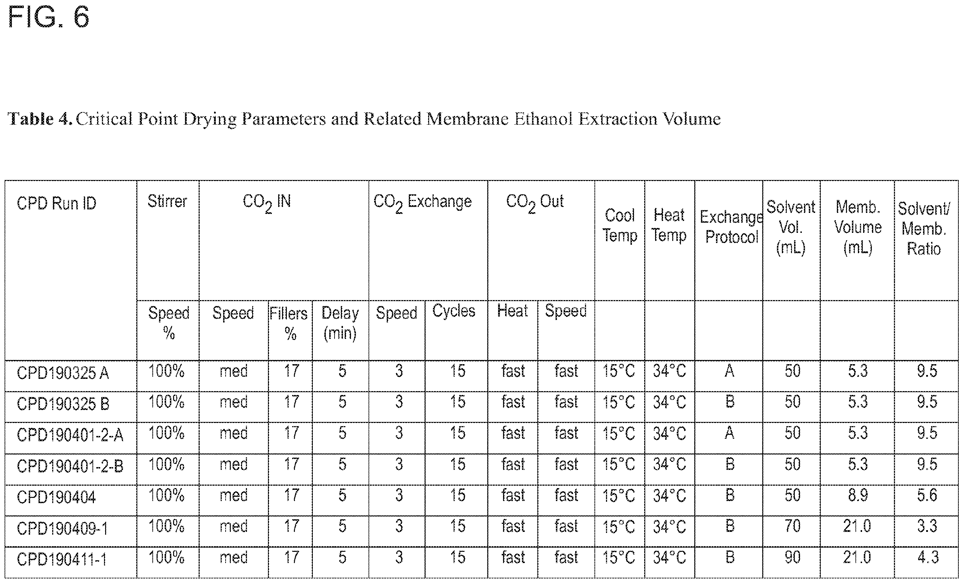

Results, as shown in Fig. 5A - 5D, reveal that when the water in the membranes was extracted (at fixed membrane-to-ethanol solution ratio (1 : 9.5) that is listed in Fig. 6, Table 4), both protocols were efficient in removing water and resulted in dry membranes with consistent properties over a 90-day storage test.

### Example 4 - Critical point drying of epoxy-containing membranes that were exchanged with different membrane-to-alcohol solution ratios

Ideally, it is preferable to minimize the amount of alcohol needed for efficient water extraction, so the associated cost, handling, and waste problems can be mitigated, especially for large-scale operations. To demonstrate the flexibility of the CPD method for drying epoxy-containing membranes while preserving their wet state characteristics, several membranes in roll format were extracted following protocol B while using different membrane-to-solution ratios (Fig. 6, Table 4: CPD190404, CPD190409-1, and CPD190411-1).

The results (Fig. 4A - 4D) indicate that it is possible to successfully prepare membranes for critical point drying if exchanged with gradient alcohol solutions and at membrane-to-solution ratios that can range from 5.6 to as low as 3.3. In fact, ratio of 9.5 is also included in effective range as the results in early experiments (that explored protocols A&B, Fig. 5A - 5D) showed that critical point drying has indeed produced storage-stable epoxy membranes at that ratio, which continued to possess the same performance characteristics, long after being dried.

### Example 5 - Critical point drying of several epoxy-containing membranes of various permeabilities

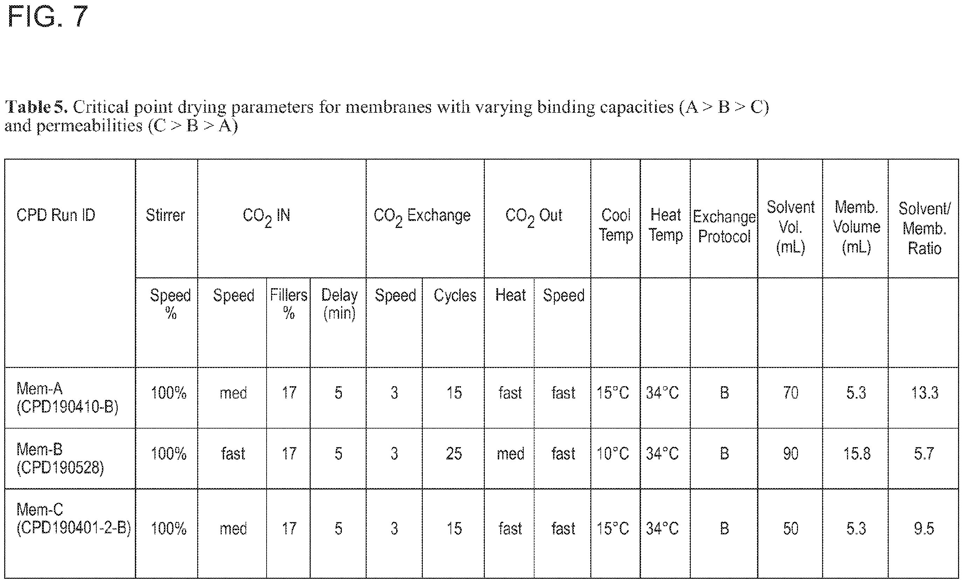

For any specific application, and particularly for bio-separation, tailoring the membrane structure, porosity, and permeability is an important part of developing a successful chromatographic platform, and it is important to demonstrate the capability of critical point drying technique to preserve the wet-state performance of various membranes with different characteristics. In this example, several epoxy-containing membranes of different permeability and different binding capacities (when conjugated) were exchanged (protocol B, Table 2) and critical point dried, using the process parameters listed in Table 5 (Fig. 7).

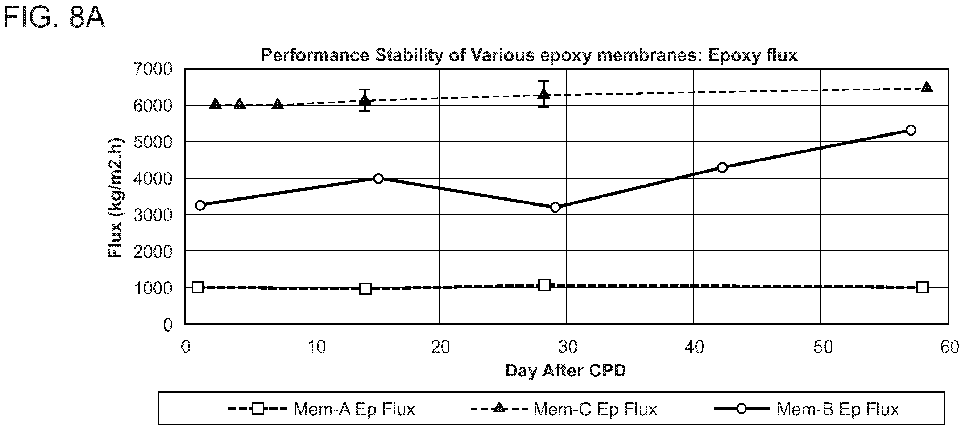

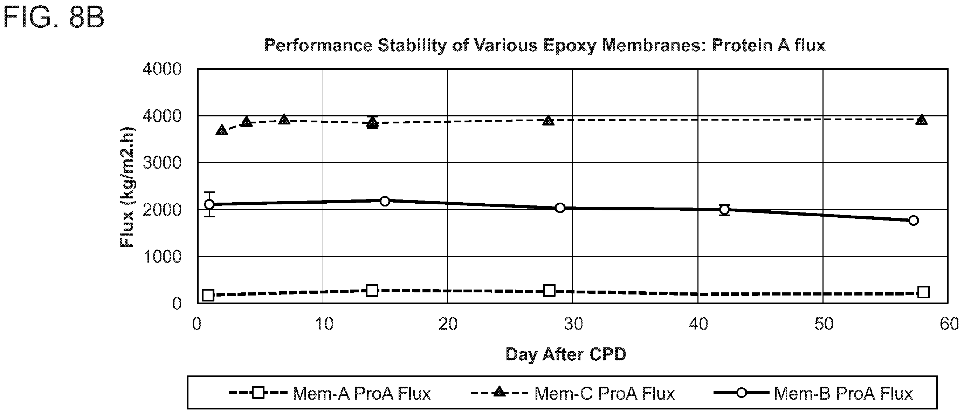

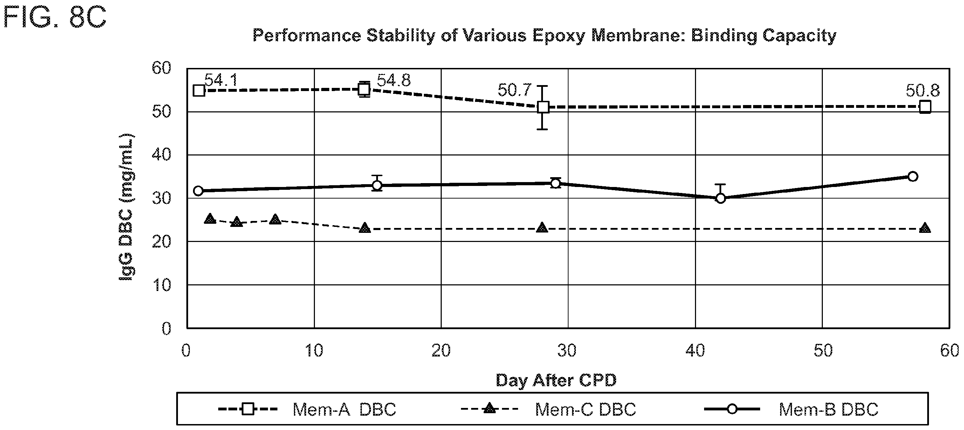

Again, the results (Fig. 8A - 8C) showed that the CPD process preserved membrane properties over extended storage times (binding capacities were measured after a subsequent Pro A conjugation step). Despite the differences in their performance characteristics, all critical point dried membranes maintained their initial permeability and binding capacity levels (after conjugated) during storage.

### Example 6 - Effect of critical point drying on membrane porosity compared to other drying methods

To demonstrate the effect of CPD method on membrane pore size and distribution, compared to oven drying, membrane samples were dried following various methods and porometry testing was carried out to follow changes in pores size.

Epoxy membrane samples were exchanged with ethanol, using protocol B (Table 2), then the membranes were dried using the following methods: A) Critical point drying; B) Membrane was soaked in water then dried in oven at 50 °C for 10 min; and finally C) Membrane was dried directly from ethanol in oven at 50 °C for 10 min.

For the ethanol wetted exchange sample serving as a non-dried reference, the membrane coupon was exchanged in 5-10 mL Porofil solution in a beaker for ~ 10-15 min to exchange ethanol with Porofil test fluid prior to testing. For all other dried samples (A, B, and C), a small disc of each membrane (18 mm dia.) was soaked in Porofil solution for at least 10 min prior to testing (as outlined in method section).

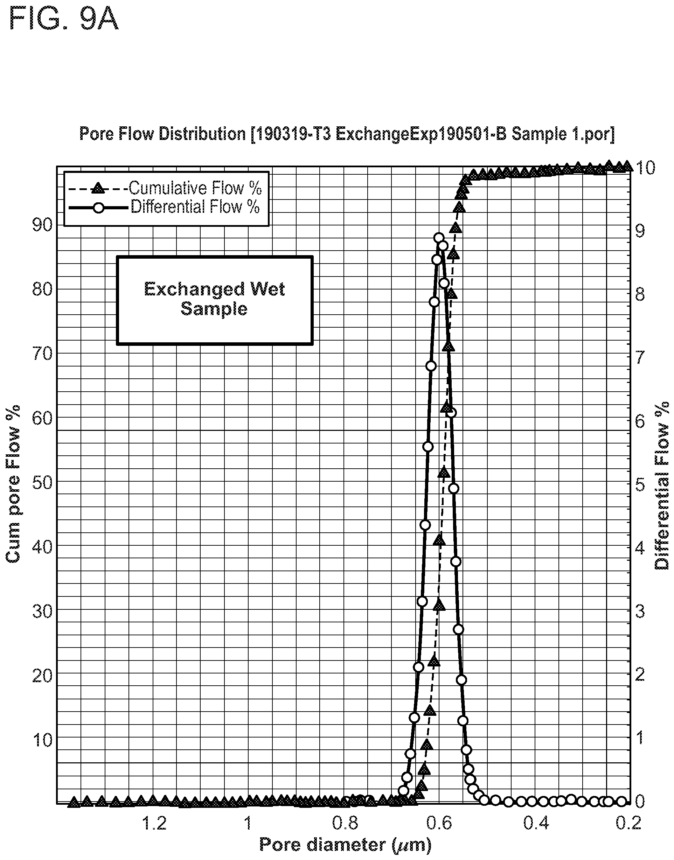

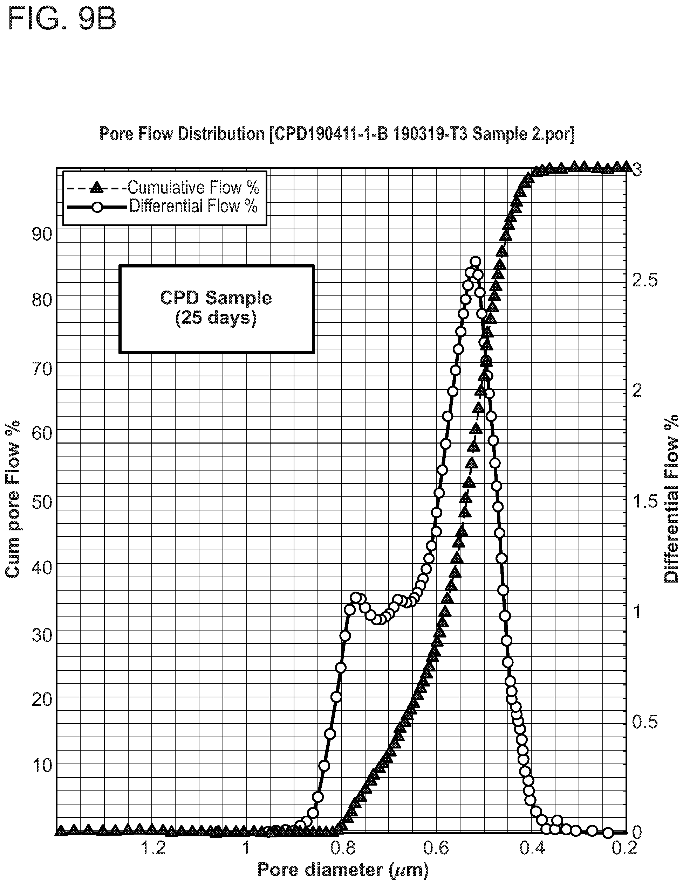

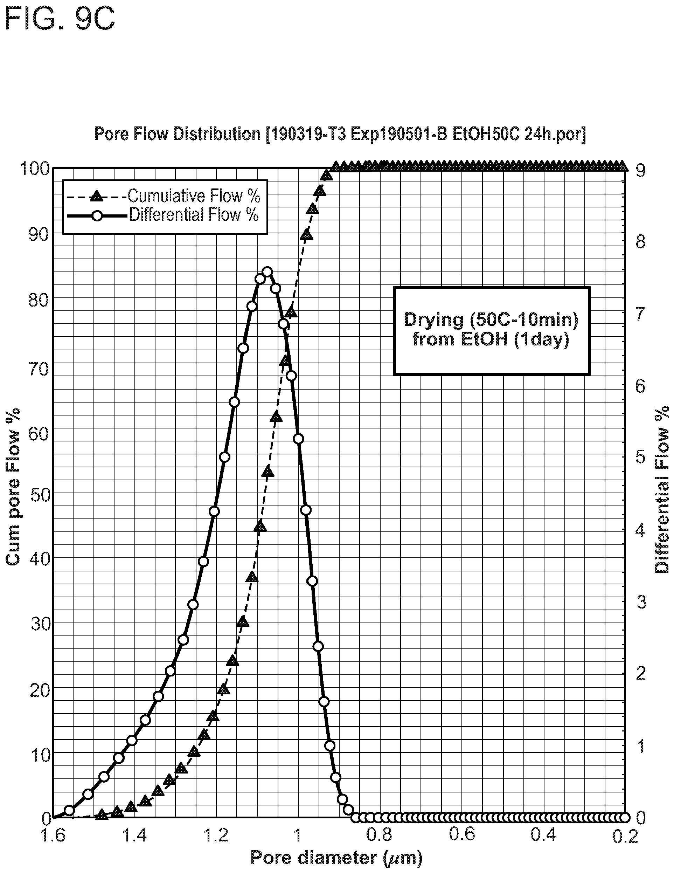

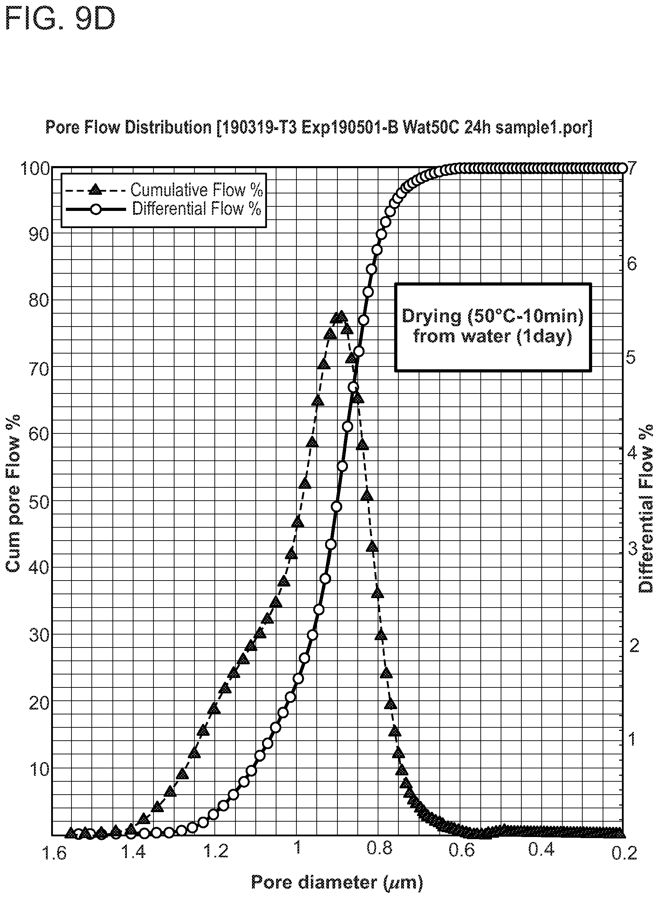

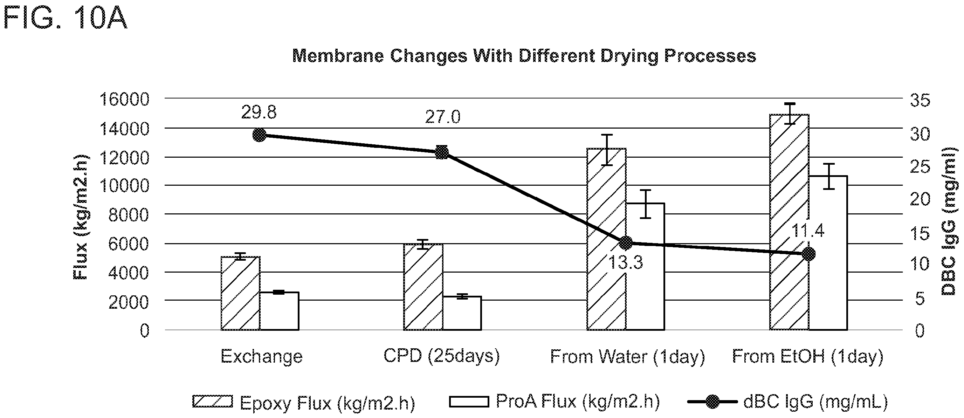

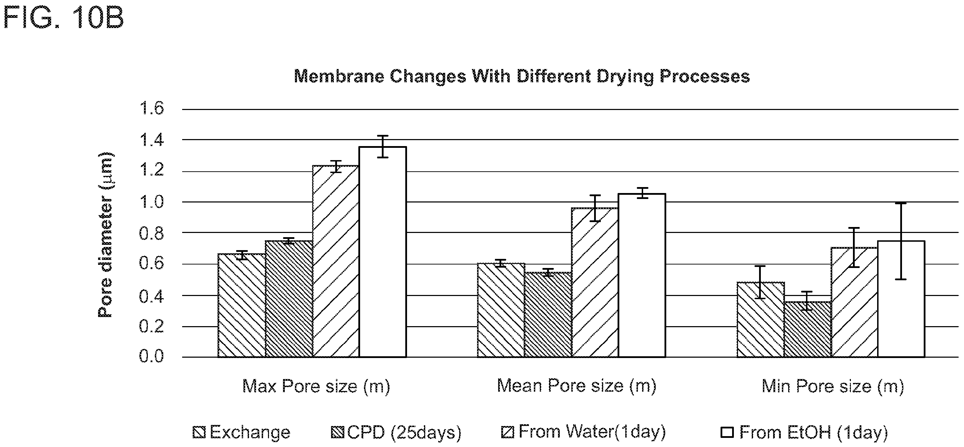

The results show that the ethanol wetted control membrane has a relatively narrower pore size distribution, compared to all of the dried samples. However, the changes in pore size and pore size distribution was much less significant for the CPD membrane sample compared to the oven-dried samples, whether from water or ethanol (Fig. 9A - 9D). The difference noted in membrane pore size characteristics generally correlated with membrane performance characteristics, namely smaller pore sizes correlate with lower membrane flux and higher binding capacity of the Protein A conjugated form (Fig. 10A and 10B).

### Example 7 - Surface area of critical point dried epoxy membrane compared to oven drying

The surface area of any membrane plays an important role in determining its interaction extent with targeted molecules in mobile phase, as most adsorption processes, especially in bio-separation, take place at the interface. Larger accessible surface area in general means larger binding capacity where chances of biomolecules to interact with functional surfaces, whether through ionic, hydrophobic, or bio-affinity interactions increase proportionally with the increase in surface area.

In this example, multiple samples of epoxy-containing membranes that have been dried using critical point drying method were analyzed for total surface area using the BET test. In addition, an oven-dried sample was also examined to compare the surface area of both types of membranes and the effect of drying process.

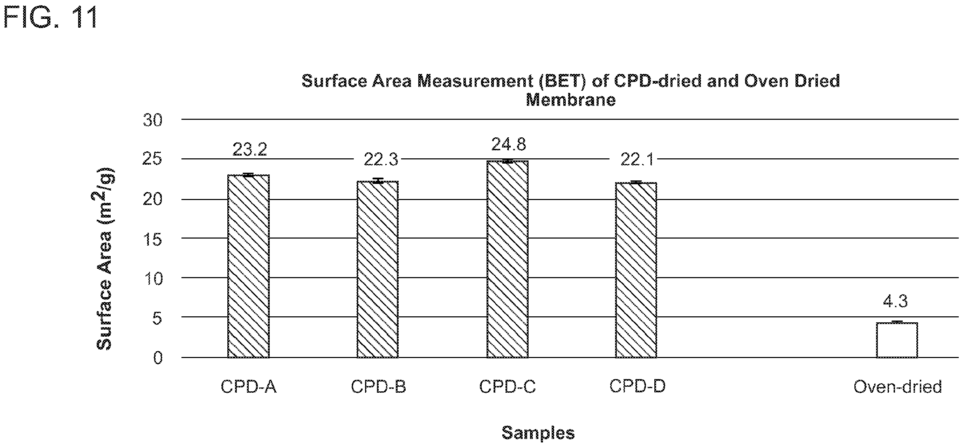

The surface areas (Fig. 11) of several CPD membranes were substantially larger than the oven dried sample, with the CPD membrane surface areas ranging from 22-25 m²/g, compared to only 4.3 m²/g for the oven dried membrane. This demonstrates that the CPD process produces a significantly larger total surface area than oven drying which very likely results in significantly higher protein binding capacity (a surface-limited process). Presumably, gel collapse with simple oven drying significantly reduces total membrane surface area and related binding capacity.

Critical point drying (from liquid CO₂) has proven to be a suitable process for drying membranes in order to preserve their wet state properties and maintain membrane properties over long storage times. While the permeability and the binding capacity of the conjugated form of such membranes change drastically when dried from water or ethanol, even at relatively mild temperatures, their counterparts which were dried using critical point drying possessed permeabilities and binding capacities similar to their wet from. It was shown through various examples that critical point drying technique can reduce drying effect on membrane gel pore size and result in membranes with high surface area, compared to other methods.

### Example 8 - Critical point drying of Protein A membrane in comparison to regular drying

In this example, typical CPD method was used to dry a Protein A conjugated membrane and the resultant materials were compared to other drying methods for Protein A membrane (oven drying at 40 °C for 20 min).

Protein A membrane was made by conjugating Protein A ligand to epoxy-containing membrane(s) (in disc format), following the conjugation method outlined in Example 1, Membrane Material section. Membrane coupons were tested before drying to determine their binding capacity and their flux to establish an undried baseline.

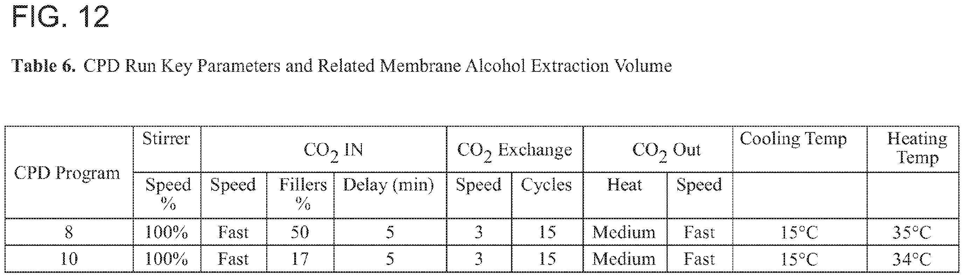

In the second stage, the membrane discs were exchanged using protocol C, a typical gradient exchange system that uses alcohol solutions, as outlined in Table 3 (Fig. 2). In the third stage, the membrane discs were loaded into the holder using screens discs of same diameter as separators, to promote better mixing and exchanging efficiency, then the holder with the membrane discs/screens stack was loaded into the CPD instrument and CPD process proceeded as described in the experimental section (Fig. 12; Table 6).

To demonstrate the contrast between a CPD approach and the typical drying method on Protein A membrane and the effect of both processes on membrane stability, additional samples of Protein A membrane were dried in the oven at 40°C for 20 min. Both materials (critical point dried and oven-dried) were examined and their binding capacity (dBC) and permeability (flux) were determined and compared.

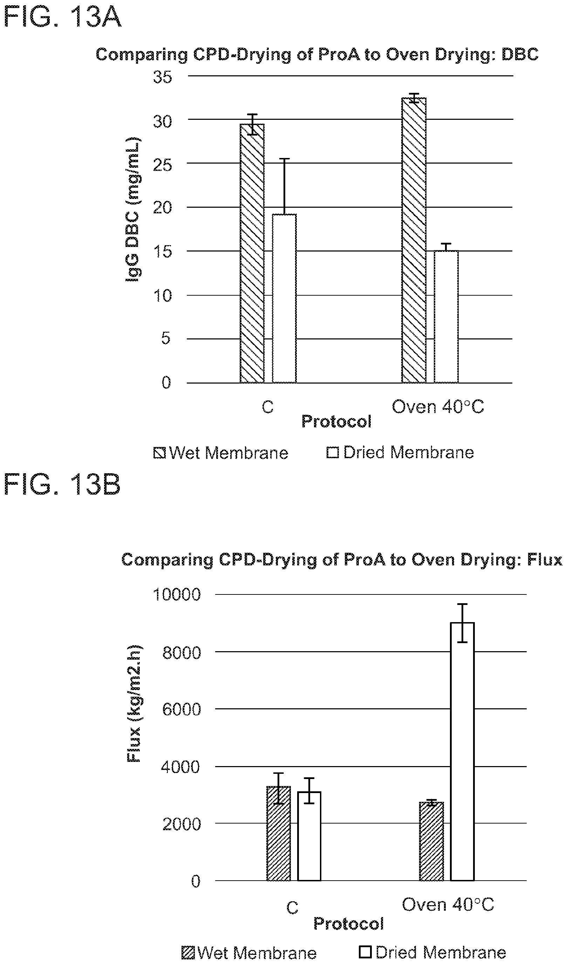

The results (Fig. 13A and 13B) show clearly how the CPD method preserved the membrane permeability as the flux of the membrane showed only minor changes, while the oven-dried sample flux has a remarkable increase over 1 day after drying. On the other hand, the results also show the loss of binding capacity of the bio-affinity membrane, which suggest that while typical exchanging protocol with only alcohol gradient did not impact the membrane platform structure, it did affect negatively the ligand and caused bioactivity loss as the dBC of the membrane decreased significantly.

### Example 9 - Critical point drying of Protein A membrane using buffer/alcohol exchanging protocols

In this example, the bioaffinity membrane was dried using a modified exchange protocol that included a long soaking step with a buffer solution with the aim to providing counter ions to the protein in order to increase the stability of the protein when exposed to the organic phases (both the alcohol solution and liquid CO₂), and the high pressure during CPD process. Two protocols, namely Awb3 and Awb7 were developed, where the first step of both protocols is a relatively long soak of membrane in a high salt buffer solution (5X and 10X of PBS, respectively), as outlined in Table 3 (Fig. 2).

In addition to the buffer soak step, the developed protocols avoid exposing the ligand to organic solutions that have relatively large amount of water, as intermediate mixtures of water/organic solutions may increase the risk of ligand denaturation and resultant loss of bioactivity. Therefore, the buffer step in both protocols was followed by sharp increases in organic fluid content from 0% to 90% in the following two steps. In general, proteins may be more stable in organic solutions of low water content. The CPD proceeds as previously described (Fig. 12; Table 6).

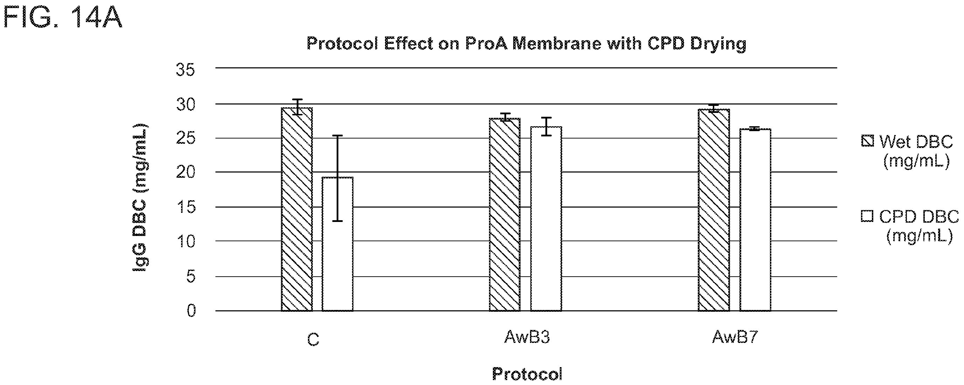

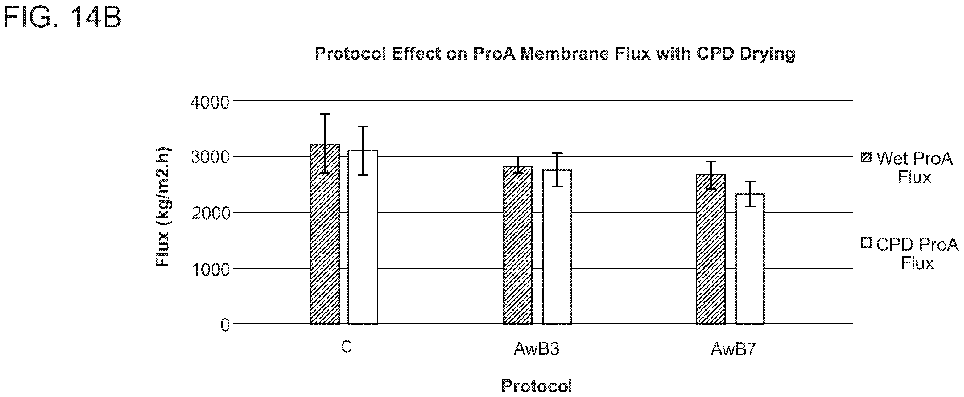

The results (Fig. 14A and 14B) demonstrated that both exchange protocols were able to produce CPD membrane of comparable properties to the wet membrane, especially binding capacity. This is an obvious contrast to Protocol C where the dBC of the dried membrane has dropped significantly.

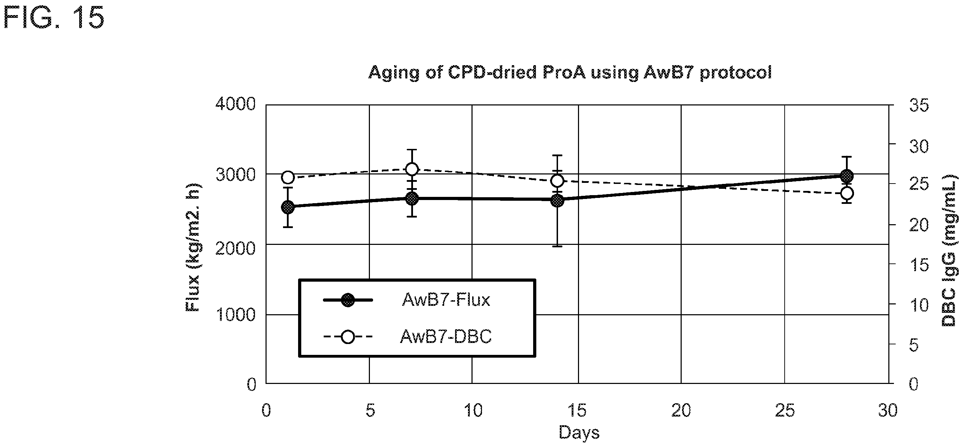

Moreover, the stability of the CPD Protein A membrane exchanged with protocol AwB7 was examined by testing the performance of the membrane after dry storage for selected periods. As demonstrated in Fig. 15, the dried membrane had generally stable performance over the testing period.

### Example 9A - Critical point drying of Protein A membrane using buffer/alcohol exchange protocol (AwB3)

The interleafed membrane roll was made as outlined in previous section, then it was exchanged from aqueous solution into an essentially pure ethanol solution using the developed protocol AwB3 (Table 7) that includes a buffer exchange step at the beginning of the exchange process. The membrane roll was simply placed in the designated amount of solution in a 200mL beaker then and was exchanged over the designated time with agitation every 2-3 minutes. At the end of every step, the solution was decanted, and fresh amount of the solution was added, following the composition outlined in the table. At the end of the exchange process the membrane roll was transferred into the CPD instrument pre-cooled chamber and the process proceeded following the designated program in auto-mode, with pre-defined key parameters, as shown in (Table 8).

**Table 7. Alcohol Exchange Protocol AwB3**

| Step | Solvent | Solven t% | Co-solvent or Buffer | Co-solven t % | Membran e Vol (mL) | Solution/ Membrane Ratio (mL/mL) | Soakin g Time (min) |
|---|---|---|---|---|---|---|---|
| 1 | anhydrou s Alcohol | 0 | 5X PBS | 100 | 30.2 | 4.6 | 20 |
| 2 | | 90 | H₂O | 10 | 30.2 | 4.6 | 10 |
| 3 | | 90 | H₂O | 10 | 30.2 | 4.6 | 10 |
| 4 | | 100 | - | 0 | 30.2 | 4.6 | 10 |
| 5 | | 100 | - | 0 | 30.2 | 4.6 | 10 |

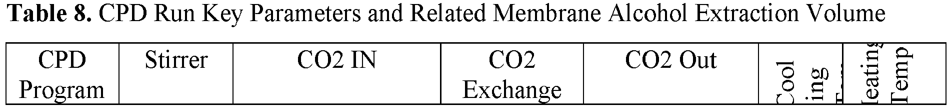

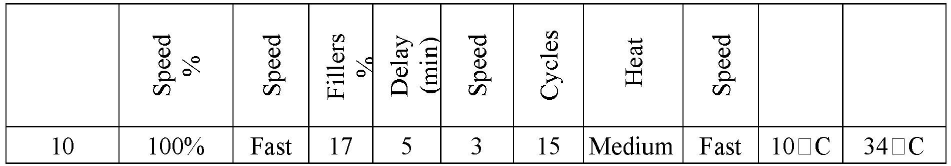

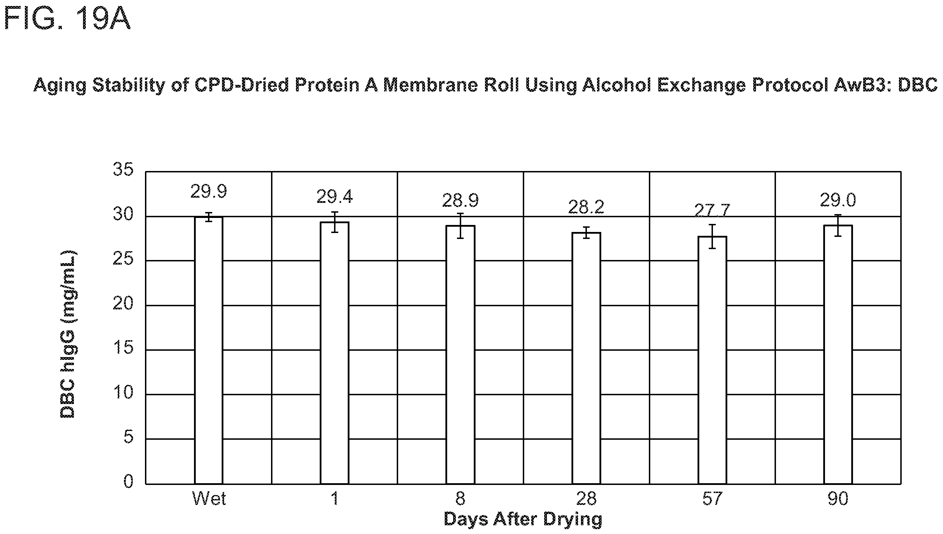

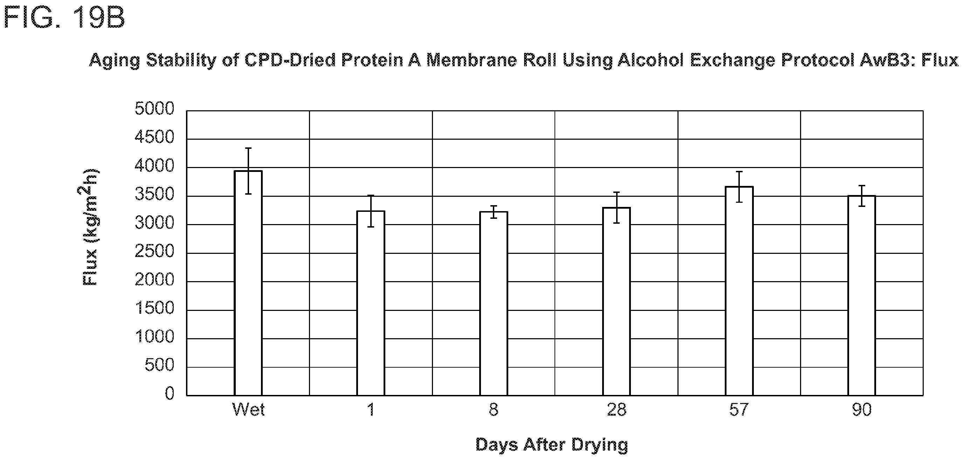

The results, as shown in Fig. 19A and 19B, demonstrate the success of the drying process in preserving the membrane binding capacity and permeability. They also show clearly the stability of the dried membrane over 90 days of storage, as the binding capacity and the membrane permeability were within membrane usual variability and were close to the initial membrane status.

### Example 9B - Critical point drying of Protein A membrane using modified buffer/alcohol exchange protocol (AwB3-SV)

In this example, the membrane interleafed roll was exchanged using a modified (short version) protocol, as outlined in Table 9 (AwB3-SV), where the membrane was exchanged first with buffer (5X PBS) then went through limited alcohol exchange process that is comprised of 2 steps, instead of the usual 4 steps in the original AwB3 protocol.

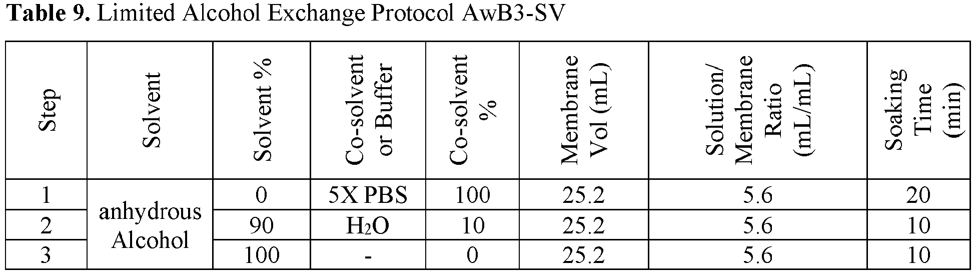

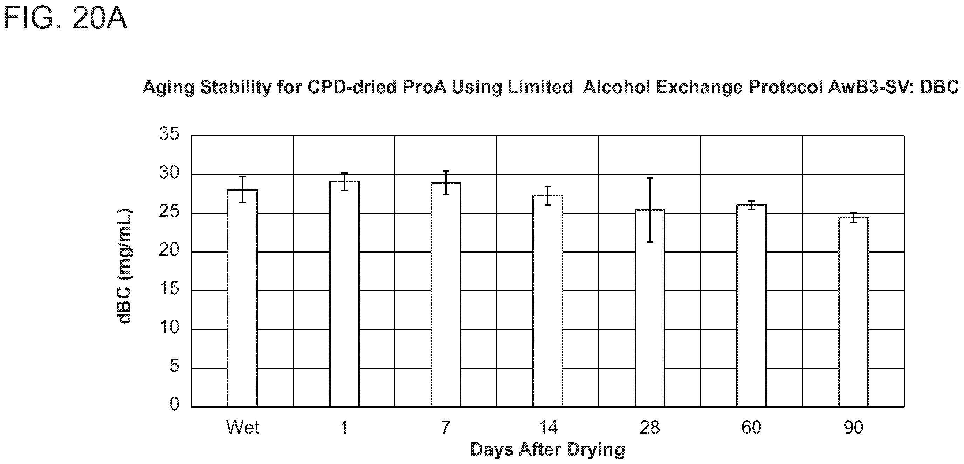

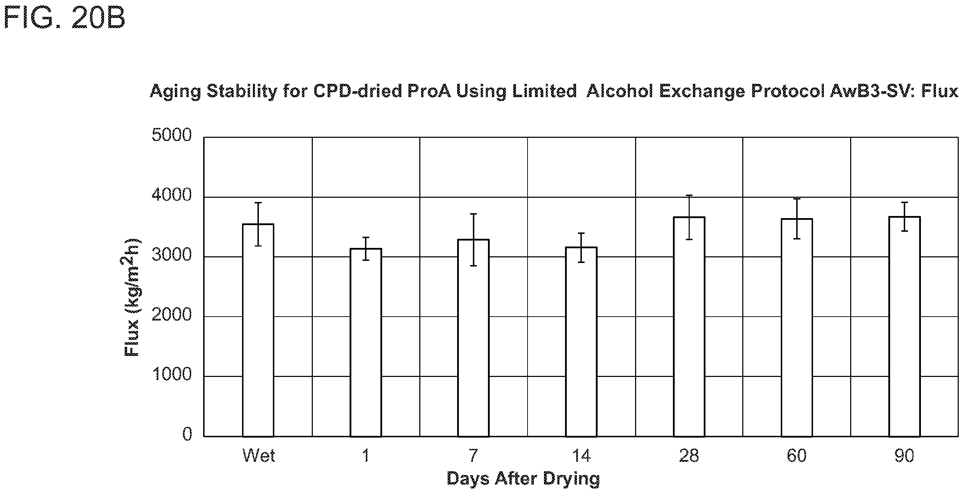

The results, as shown in Fig. 20A and 20B showed that despite using a limited exchange protocol, it was still possible to produce CP-dried membrane that was stable during dry storage. The membranes went through marginal changes in capacity and flux within the first two weeks then stabilized over the rest of the testing time period.

### Example 9C - Critical point drying of Protein A membrane after storage in biostatic solution (20% alcohol in water)

To illustrate the flexibility of this drying approach, a protocol that included a first storage period in biostatic solution(s) before proceeding to the alcohol exchange and CP drying processes. In this case, the protocol was split into two sub-protocols: 1) the step where the membrane is prepared and transferred into biostatic storage solution, and 2) alcohol exchange step where a simplified shorter protocol is applied to exchange the storage solution for essentially pure alcohol in preparation for the CP drying process. One protocol, as outlined in (TP1-AWOB1, Table 10), shows the process step where the membrane is directly transferred and exchanged in 20% alcohol solution then removed and stored in sealed PP bag for 1 (or 7) days. In alternative protocol (TP2-AWOB1, Table 11), the membrane roll is exchanged with buffer solution (5X PBS) first to allow sufficient ionic group incorporation, after which the membrane is exchanged in 20% alcohol (in water) then stored for a short period of time (1day for the first set, or 7days for the second set). At the end of each experiment storage period, the membrane is subjected to an exchange protocol to replace the primarily aqueous storage solutions with alcohol in preparation for the CP drying process.

**Table 10. Exchange Protocol with Biostatic Storage Period TP1-AWOB1**

| Transition Preparation Protocol: TP-1 | | | | | | | |
|---|---|---|---|---|---|---|---|
| Step | Solvent | Solvent % | Co-solvent or Buffer | Co-solvent % | Membrane Vol (mL) | Solution/ Membrane Ratio (mL/mL) | Soaking Time (min) |
| 1 | anhydrous Alcohol | 20 | H₂O | 140 | 20.2 | 6.9 | 10 |

| **Storage Period: 1 OR 7 days** | | | | | | | |
|---|---|---|---|---|---|---|---|
| Alcohol Exchange Protocol-AWOB-1 | | | | | | | |
| Step | Solvent | Solvent % | Co-solvent or Buffer | Co-solvent % | Membrane Vol (mL) | Solution/ Membrane Ratio (mL/mL) | Soaking Time (min) |
| 1 | anhydrous Alcohol | 90 | H₂O | 10 | 20.2 | 6.9 | 10 |
| 3 | | 100 | - | 0 | 20.2 | 6.9 | 10 |
| 4 | | 100 | - | 0 | 20.2 | 6.9 | 10 |

**Table 11. Exchange Protocol with Buffer Exchange and Biostatic Storage Period TP2-AWOB1**

| Transfer Preparation Protocol: TP-2 | | | | | | | |
|---|---|---|---|---|---|---|---|
| Step | Solvent | Solvent % | Co-solvent or Buffer | Co-solvent % | Membrane Vol (mL) | Solution/Membrane Ratio (mL/mL) | Soaking Time (min) |
| 1 | anhydrous Alcohol | 0 | 5X PBS | 100 | 25.2 | 5.6 | 20 |
| 2 | | 20 | H₂O | 80 | 25.2 | 5.6 | 10 |

| **Storage Period: 1 OR 7 days** | | | | | | | |
|---|---|---|---|---|---|---|---|
| Alcohol Exchange Protocol-AWOB-1 | | | | | | | |
| Step | Solvent | Solvent % | Co-solvent or Buffer | Co-solvent % | Membrane Vol (mL) | Solution/Membrane Ratio (mL/mL) | Soaking Time (min) |
| 1 | anhydrous Alcohol | 90 | H₂O | 10 | 25.2 | 5.6 | 10 |
| 3 | | 100 | - | 0 | 25.2 | 5.6 | 10 |
| 4 | | 100 | - | 0 | 25.2 | 5.6 | 10 |

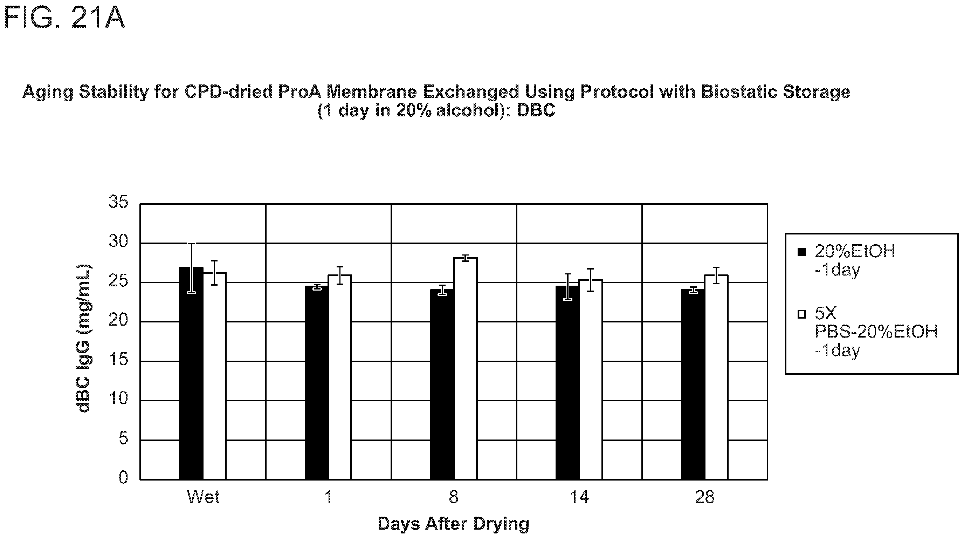

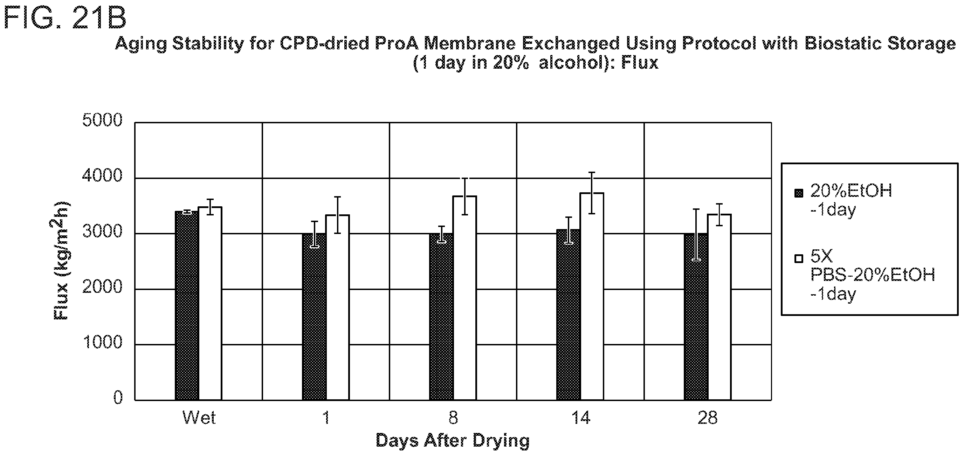

The results of the first set (Fig. 21A and 21B), where the membranes were stored for 1 day using two alcohol exchange protocols, show that the CP drying process produced membranes that closely resemble the original wet form, and they were essentially stable with dry storage over the testing period.

It is notable that although the presence of buffer exchange step tends to produce membrane with marginal increase of permeability, the absence of the buffer exchange step in the first protocol did not impact negatively the drying process outcome. This may result from the short storage time used here (1day), and that the alcohol percentage in the exchange solution were either very low (<20%) or very high (90 & 100%). These concentrations were deliberately chosen to avoid ligand structural changes as proteins tend to be more stable in either predominantly aqueous solutions or neat organic solvents.

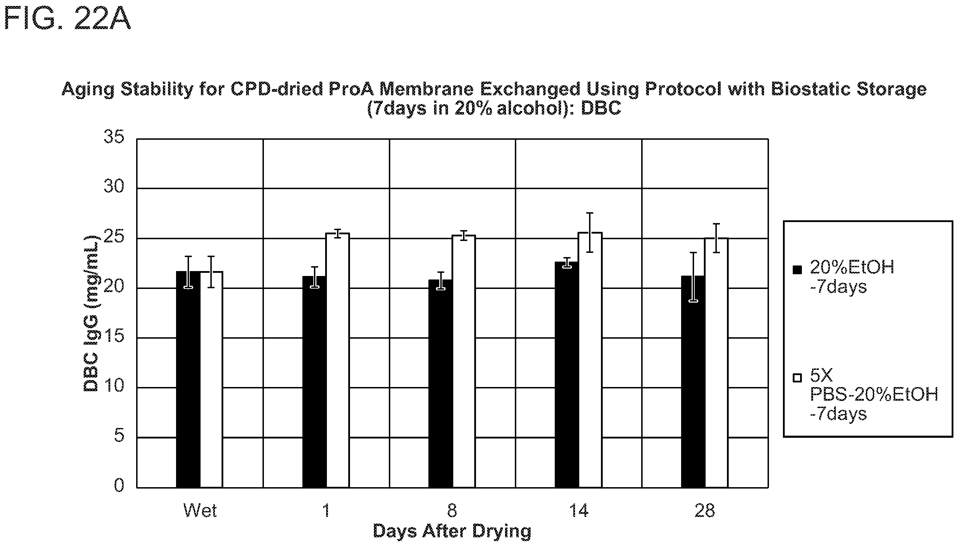

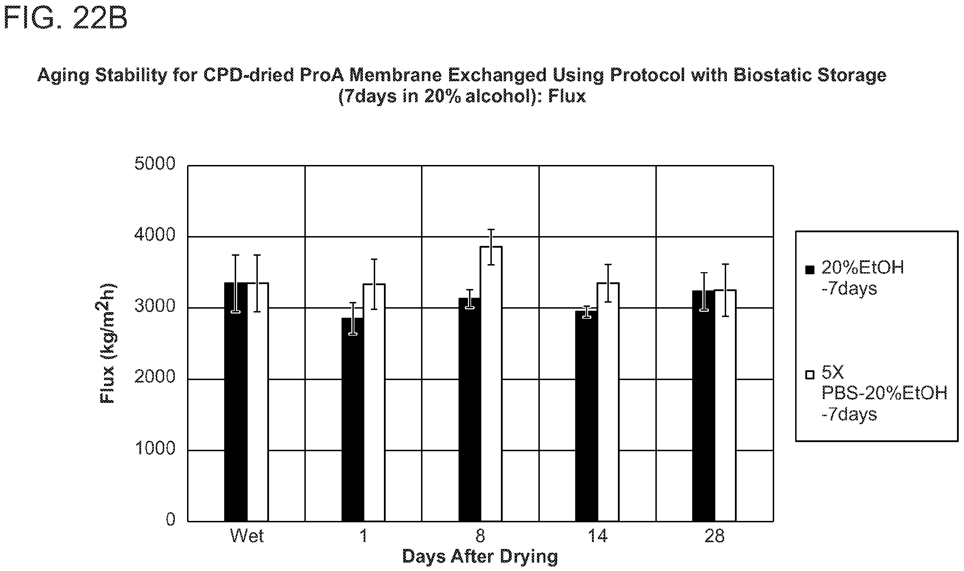

On the other hand, the results of the second set (Fig. 22A and 22B), where the membranes were stored in a 20% alcohol solution for 7 days, shows how the inclusion of the buffer exchange step prior to 20% alcohol storage (protocol TP2-AWOB 1) resulted in increased dried membrane binding capacity, but no clear effect on membrane permeability (as the measured by flux).

### Example 9D - Critical point drying of Protein A membrane using exchange protocols with storage in 16% alcohol in 20 mM phosphate buffer (pH 7.5)

In this example, another biostatic storage solution (16% alcohol in 20 mM phosphate buffer, pH 7.5) was used to store membranes for 7 days before alcohol exchange and CP drying, as shown in Table 12 that outlines protocol TP3-AWOB1, and in Table 13 that outlines the alternative protocol TP4-AWOB 1, which includes a buffer exchange step (5X PBS).

**Table 12. Exchange Protocol with Biostatic Storage Period in 16% alcohol in 20mM Phosphate (TP3-AWOB1)**

| Transfer Preparation Protocol: TP-3 | | | | | | | |
|---|---|---|---|---|---|---|---|
| Step | Solvent | Solvent % | Co-solvent or Buffer | Co-solvent % | Membrane Vol (mL) | Solution/Membrane Ratio (mL/mL) | Soaking Time (min) |
| 1 | anhydrous Alcohol | 16 | 20 mM Phosphate, pH7.5 | 140 | 20.2 | 6.9 | 10 |

| **Storage Period: 7 days** | | | | | | | |
|---|---|---|---|---|---|---|---|
| Alcohol Exchange Protocol-AWOB-1 | | | | | | | |
| Step | Solvent | Solvent % | Co-solvent or Buffer | Co-solvent % | Membrane Vol (mL) | Solution/Membrane Ratio (mL/mL) | Soaking Time (min) |
| 1 | anhydrous Alcohol | 90 | H₂O | 10 | 25.2 | 5.6 | 10 |
| 3 | | 100 | - | 0 | 25.2 | 5.6 | 10 |
| 4 | | 100 | - | 0 | 25.2 | 5.6 | 10 |

**Table 13. Exchange Protocol with Buffer Exchange and Biostatic Storage Period in 16% alcohol in 20mM Phosphate (TP4-AWOB1)**

| Transfer Preparation Protocol: TP-4 | | | | | | | |
|---|---|---|---|---|---|---|---|
| Step | Solvent | Solvent % | Co-solvent or Buffer | Co-solvent % | Membrane Vol (mL) | Solution/ Membrane Ratio (mL/mL) | Soaking Time (min) |
| 1 | anhydrous Alcohol | 0 | 5X PBS | 100 | 25.2 | 5.6 | 20 |
| 2 | | 16 | 20 mM Phosphate, pH7.5 | 84 | 25.2 | 5.6 | 10 |

| **Storage Period: 7 days** | | | | | | | |
|---|---|---|---|---|---|---|---|
| Alcohol Exchange Protocol-AWOB-1 | | | | | | | |
| Step | Solvent | Solvent % | Co-solvent or Buffer | Co-solvent % | Membrane Vol (mL) | Solution/ Membrane Ratio (mL/mL) | Soaking Time (min) |
| 1 | anhydrous Alcohol | 90 | H₂O | 10 | 25.2 | 5.6 | 10 |
| 3 | | 100 | - | 0 | 25.2 | 5.6 | 10 |
| 4 | | 100 | - | 0 | 25.2 | 5.6 | 10 |

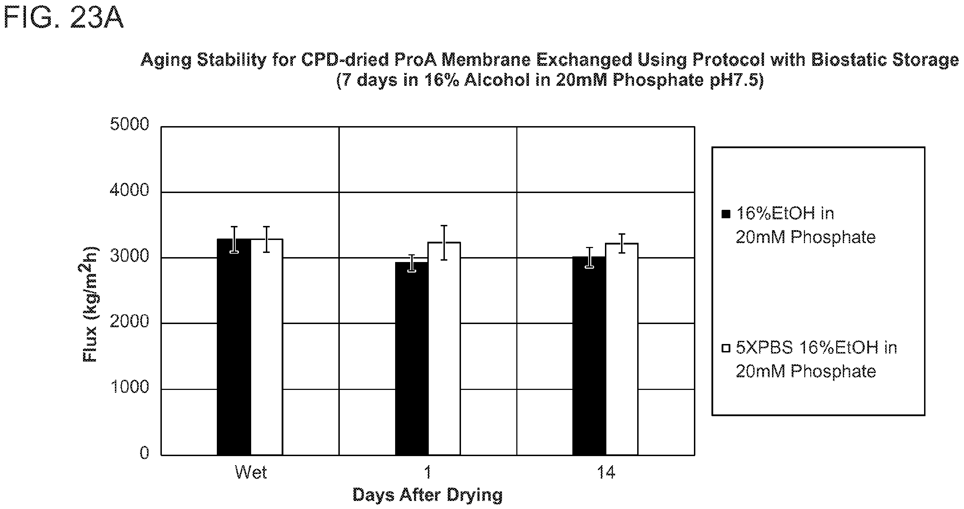

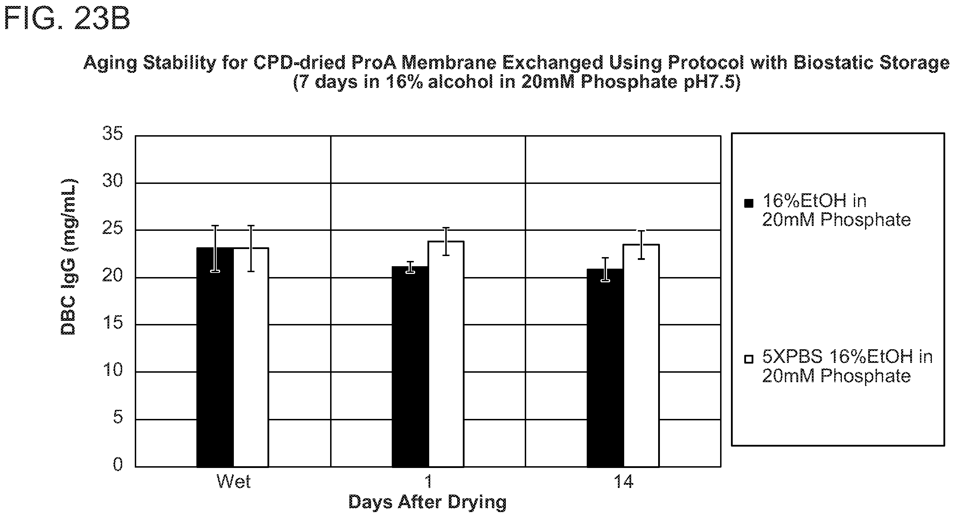

Results, as shown in Fig. 23A and 23B, demonstrated again the ability of the CPD process to produce dry membranes that resemble the wet form. While the impact of the drying process was very minimal, for both exchange protocols, the presence of the buffer exchange step in the second protocol had slightly better outcome for the binding capacity.

### Example 10 - Critical point drying of Protein A membrane using salt/alcohol exchanging protocol

In this example, the use of salt instead of buffer as a source of ions, particularly multivalent cations such as divalent calcium cation, to modify the solvent exchange system prior to CPD process was demonstrated. Divalent calcium cations are well known to impact protein structure through coordination bonds that serve to stabilize biomolecule structure in solution. In addition, calcium salt has good solubility in anhydrous alcohols solution, an advantage that PBS buffer does not have. This would allow a continued supply of calcium cations to the membrane during the last few steps of the exchange protocol to ensure the cations remain in contact with the membrane before moving to CPD process.

Similar to the previous example, Protein A membrane discs were made by functionalizing epoxy-containing membranes, then exchanged similarly but following AwB9 protocol (Table 3; Fig. 2) that has calcium chloride solution (2 M) in water instead of PBS buffer solutions, then critical point dried as outlined previously using key parameters listed in Table 6 (Fig. 12; Program 8).

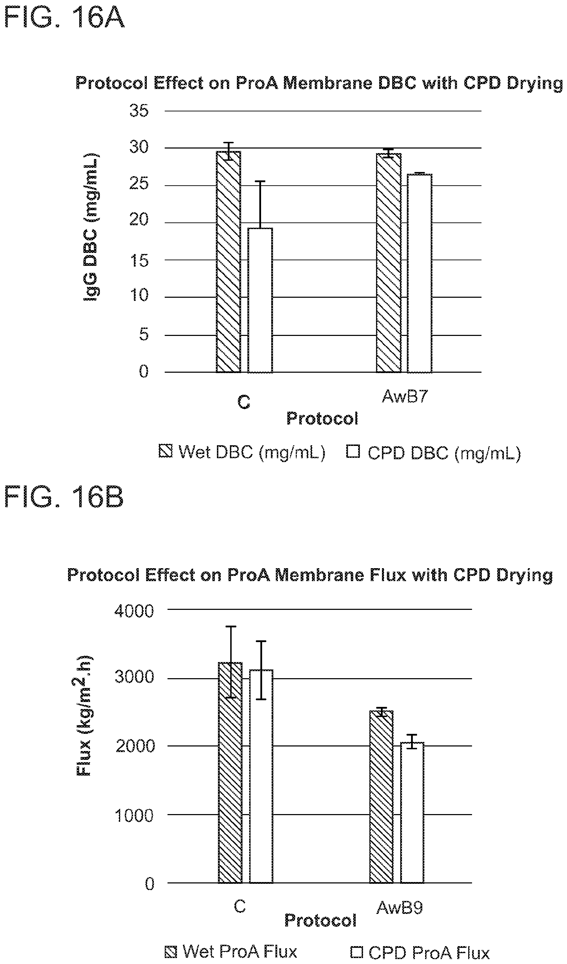

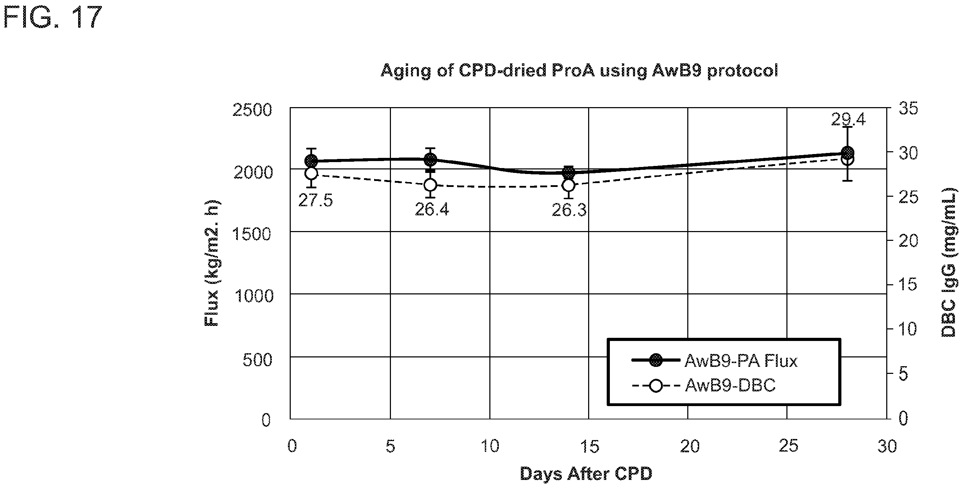

The results (Fig. 16A and 16B) demonstrated the positive effect of incorporating calcium cation in the exchange protocol (AwB9) compared to Protocol C, particularly on binding capacity that remains essentially unchanged, and only a minor initial drop in membrane flux. Long term dry storage testing showed that the membrane flux and binding capacity were consistent over the selected test period (Fig. 17).

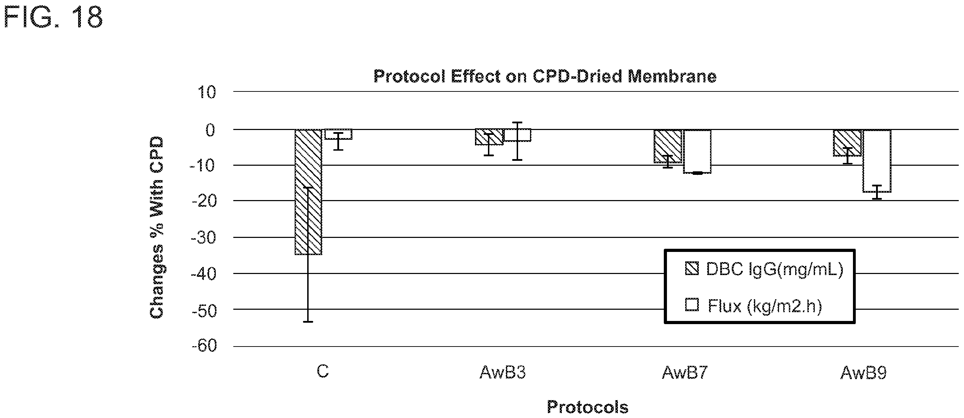

When comparing the results of using a salt-incorporated exchange protocol and buffer-incorporated protocol versus plain gradient protocol with no additives (Fig. 18), it was clear that adding salt and/or buffer soaking steps mitigated the impact of the exchange process on membrane binding capacity and permeability. This suggests that the CPD technique described here may be employed to effectively treat and dry delicate porous structures and immobilized ligands. Critical point drying, using the modified exchange protocols, has proven to be a successful method for drying bio-affinity membranes where a bioligand is covalently conjugated to the membrane surface. The critical point-dried materials have chromatographic performance similar to their pre-dried form and their performance and characteristics were consistent when stored dry for extended times.

Modifications of typical exchange protocols were necessary for the described CPD process: Incorporating buffer and salt exchange steps in the protocol allows the media to tolerate the negative effects resulting from exposure to both exchange and drying process fluids and conditions.

This invention provides the opportunity to use CPD approach to handle and dry a wide range of materials that contain a sensitive ligand and robust matrix or membrane, or robust bioligand on labile matrix or membrane, or sensitive bioligand immobilized on labile matrix or membrane. In all cases, the CPD process and related exchange protocols must be modified to accommodate the sensitivity of either the bioligand or the platform on which the ligand is immobilized.

## Claims

1. A method of critical point drying a composite material, comprising the steps of:
a. providing a composite material comprising:
i. a support member, comprising a plurality of pores extending through the support member; and
ii. a macroporous cross-linked gel, wherein the macroporous cross-linked gel comprises a polymer formed from a reaction of one or more polymerizable monomers with one or more cross-linkers; the macroporous cross-linked gel is located in the pores of the support member; and said macropores of the macroporous cross-linked gel are smaller than the pores of the support member;
wherein the composite material is wetted with an aqueous solution;
b. exchanging the aqueous solution with a polar organic solvent, such that the composite material is wetted with the polar organic solvent;
c. contacting the composite material with a supercritical fluid at a temperature and a pressure above the critical point of the supercritical fluid, wherein the gaseous and liquid phases are indistinguishable; and the polar organic solvent is miscible with the supercritical fluid; and
d. reducing the pressure;
thereby critical point drying the composite material.

2. The method of claim 1, wherein
the macroporous cross-linked gel comprises a plurality of ligands that are biological molecules or biological ions.

3. The method of any one of claims 1-2, wherein the supercritical fluid is selected from the group consisting of carbon dioxide, water, methane, ethane, propane, ethylene, propylene, methanol, ethanol, acetone, and nitrous oxide.

4. The method of any one of claims 1-2, wherein the supercritical fluid is selected from the group consisting of carbon dioxide, methane, ethane, propane, ethylene, propylene, methanol, ethanol, acetone, and nitrous oxide.

5. The method of any one of claims 1-2, wherein the supercritical fluid is carbon dioxide.

6. The method of any one of claims 1-5, wherein one or more parameters selected from the group consisting of total amount of supercritical fluid relative to sample volume, number of purge cycles, temperature during contact with the supercritical fluid, the exchange rate with the supercritical fluid, the temperature during the evaporation step, the heating rate, and the rate of the pressure reduction are adjusted.

7. The method of any one of claims 1-6, wherein the polar organic solvent is selected from the group consisting of acetone, acetonitrile, ammonia, t-butanol, n-propanol, ethanol, methanol, and acetic acid.

8. The method of any one of claims 2-7, wherein the biological molecules or biological ions are selected from the group consisting of albumins, lysozyme, viruses, cells, γ-globulins of human and animal origins, immunoglobulins of both human and animal origins, proteins of recombinant or natural origin including, polypeptides of synthetic or natural origin, interleukin-2 and its receptor, enzymes, monoclonal antibodies, antigens, lectins, bacterial immunoglobulin-binding proteins, trypsin and its inhibitor, cytochrome C, myoglobulin, recombinant human interleukin, recombinant fusion protein, Protein A, Protein G, Protein L, Peptide H, nucleic acid derived products, DNA of either synthetic or natural origin, and RNA of either synthetic or natural origin;
optionally wherein the biological molecules or biological ions are Protein A.

9. The method of any one of claims 1-8, wherein the support member comprises a polymeric material selected from the group consisting of polysulfones, polyethersulfones, polyphenyleneoxides, polycarbonates, polyesters, cellulose, and cellulose derivatives.

10. The method of any one of claims 1-9, wherein the composite material is a membrane.

11. The method of any one of any one of claims 1-10, wherein the composite material is in contact with one or more interleaf layers.

12. The method of claim 11, wherein the one or more interleaf layers are selected from the group consisting of screen, polypropylene, polyethylene, and paper.

13. The method of any one of claims 1-12, wherein the cross-linked gel is a neutral hydrogel, a charged hydrogel, a polyelectrolyte gel, a hydrophobic gel, a neutral gel, or a gel comprising functional groups.

14. The method of any one of claims 1-13, wherein the macroporous cross-linked gel comprises macropores having an average size of about 10 nm to about 3000 nm.

15. The method of any one of claims 1-14, wherein the pores of the support member have an average pore diameter of about 0.1 µm to about 50 µm.

## Patentansprüche

1. Verfahren zum Kritisch-Punkt-Trocknen eines Verbundmaterials, umfassend die Schritte des:
a. Bereitstellens eines Verbundmaterials, umfassend:
i. ein Trägerelement, umfassend eine Vielzahl von Poren, die sich durch das Trägerelement erstrecken; und
ii. ein makroporöses vernetztes Gel, wobei das makroporöse vernetzte Gel ein Polymer umfasst, das aus einer Reaktion eines oder mehrerer polymerisierbarer Monomere mit einem oder mehreren Vernetzern gebildet wird; das makroporöse vernetzte Gel sich in den Poren des Trägerelements befindet; und die Makroporen des makroporösen vernetzten Gels kleiner sind als die Poren des Trägerelements;
wobei das Verbundmaterial mit einer wässrigen Lösung benetzt ist;
b. Austauschens der wässrigen Lösung mit einem polaren organischen Lösungsmittel, sodass das Verbundmaterial mit dem polaren organischen Lösungsmittel benetzt ist;
c. Inkontaktbringens des Verbundmaterials mit einem überkritischen Fluid bei einer Temperatur und einem Druck oberhalb des kritischen Punkts des überkritischen Fluids, wobei die gasförmige und die flüssige Phase nicht unterscheidbar sind; und das polare organische Lösungsmittel mischbar ist mit dem überkritischen Fluid; und
d. Reduzierens des Drucks;
wodurch ein Kritisch-Punkt-Trocknen des Verbundmaterials erfolgt.

2. Verfahren nach Anspruch 1, wobei
das makroporöse vernetzte Gel eine Vielzahl von Liganden umfasst, die biologische Moleküle oder biologische Ionen sind.

3. Verfahren nach einem der Ansprüche 1-2, wobei das überkritische Fluid ausgewählt ist aus der Gruppe bestehend aus Kohlendioxid, Wasser, Methan, Ethan, Propan, Ethylen, Propylen, Methanol, Ethanol, Aceton und Distickstoffoxid.

4. Verfahren nach einem der Ansprüche 1-2, wobei das überkritische Fluid ausgewählt ist aus der Gruppe bestehend aus Kohlendioxid, Methan, Ethan, Propan, Ethylen, Propylen, Methanol, Ethanol, Aceton und Distickstoffoxid.

5. Verfahren nach einem der Ansprüche 1-2, wobei das überkritische Fluid Kohlendioxid ist.

6. Verfahren nach einem der Ansprüche 1-5, wobei ein oder mehrere Parameter, die ausgewählt sind aus der Gruppe bestehend aus Gesamtmenge an überkritischem Fluid relativ zu dem Probenvolumen, Anzahl der Purgezyklen, Temperatur während des Kontakts mit dem überkritischen Fluid, der Austauschrate mit dem überkritischem Fluid, der Temperatur während des Verdampfungsschritts, der Heizrate und der Druckreduzierungsrate, eingestellt werden.

7. Verfahren nach einem der Ansprüche 1-6, wobei das polare organische Lösungsmittel ausgewählt ist aus der Gruppe bestehend aus Aceton, Acetonitril, Ammoniak, t-Butanol, n-Propanol, Ethanol, Methanol und Essigsäure.

8. Verfahren nach einem der Ansprüche 2-7, wobei die biologischen Moleküle oder biologischen Ionen ausgewählt sind aus der Gruppe bestehend aus Albuminen, Lysozym, Viren, Zellen, γ-Globulinen menschlichen und tierischen Ursprungs, Immunglobulinen sowohl menschlichen als auch tierischen Ursprungs, Proteinen rekombinanten oder natürlichen Ursprungs, einschließlich Polypeptiden synthetischen oder natürlichen Ursprungs, Interleukin-2 und dessen Rezeptor, Enzymen, monoklonalen Antikörpern, Antigenen, Lektinen, bakteriellen Immunglobulin-bindenden Proteinen, Trypsin und dessen Inhibitor, Cytochrom C, Myoglobulin, rekombinantem menschlichen Interleukin, rekombinantem Fusionsprotein, Protein A, Protein G, Protein L, Peptid H, von einer Nukleinsäure abgeleiteten Produkten, DNA entweder synthetischen oder natürlichen Ursprungs und RNA entweder synthetischen oder natürlichen Ursprungs; optional, wobei die biologischen Moleküle oder biologischen Ionen Protein A sind.

9. Verfahren nach einem der Ansprüche 1-8, wobei das Trägerelement ein polymeres Material umfasst, das ausgewählt aus der Gruppe bestehend aus Polysulfonen, Polyethersulfonen, Polyphenylenoxiden, Polycarbonaten, Polyestern, Cellulose und Cellulosederivaten.

10. Verfahren nach einem der Ansprüche 1-9, wobei das Verbundmaterial eine Membran ist.

11. Verfahren nach einem der Ansprüche 1-10, wobei das Verbundmaterial in Kontakt mit einer oder mehreren Zwischenschichten ist.

12. Verfahren nach Anspruch 11, wobei die eine oder mehreren Zwischenschichten ausgewählt sind aus der Gruppe bestehend aus Sieb, Polypropylen, Polyethylen und Papier.

13. Verfahren nach einem der Ansprüche 1-12, wobei das vernetzte Gel ein neutrales Hydrogel, ein geladenes Hydrogel, ein Polyelektrolytgel, ein hydrophobes Gel, ein neutrales Gel oder ein Gel umfassend funktionelle Gruppen ist.

14. Verfahren nach einem der Ansprüche 1-13, wobei das makroporöse vernetzte Gel Makroporen mit einer durchschnittlichen Größe von etwa 10 nm bis etwa 3000 nm umfasst.

15. Verfahren nach einem der Ansprüche 1-14, wobei die Poren des Trägerelements einen durchschnittlichen Porendurchmesser von etwa 0,1 µm bis etwa 50 µm aufweisen.

## Revendications

1. Procédé de séchage au point critique d'un matériau composite, comprenant les étapes de :
a. production d'un matériau composite comprenant :
i. un élément support, comprenant une pluralité de pores s'étendant à travers l'élément support ; et
ii. un gel réticulé macroporeux, ledit gel réticulé macroporeux comprenant un polymère formé à partir d'une réaction d'un ou plusieurs monomères polymérisables avec un ou plusieurs agents de réticulation ; ledit gel réticulé macroporeux se trouvant dans les pores de l'élément support ; et lesdits macropores du gel réticulé macroporeux étant plus petits que les pores de l'élément support ;
ledit matériau composite étant mouillé avec une solution aqueuse ;
b. échange de la solution aqueuse avec un solvant organique polaire, de sorte que le matériau composite soit mouillé avec le solvant polaire organique ;
c. mise en contact du matériau composite avec un fluide supercritique à une température et une pression supérieures à celles du point critique du fluide supercritique, les phases gazeuse et liquide étant indifférenciables ; et ledit solvant organique polaire étant miscible au fluide supercritique ; et
d. réduction de la pression ;
ce qui permet le séchage au point critique du matériau composite.

2. Procédé selon la revendication 1,
ledit gel réticulé macroporeux comprenant une pluralité de ligands qui sont des molécules biologiques ou des ions biologiques.

3. Procédé selon l'une quelconque des revendications 1 à 2, ledit fluide supercritique étant choisi dans le groupe constitué par le dioxyde de carbone, l'eau, le méthane, l'éthane, le propane, l'éthylène, le propylène, le méthanol, l'éthanol, l'acétone et l'oxyde nitreux.

4. Procédé selon l'une quelconque des revendications 1 à 2, ledit fluide supercritique étant choisi dans le groupe constitué par le dioxyde de carbone, le méthane, l'éthane, le propane, l'éthylène, le propylène, le méthanol, l'éthanol, l'acétone et l'oxyde nitreux.

5. Procédé selon l'une quelconque des revendications 1 à 2, ledit fluide supercritique étant le dioxyde de carbone.

6. Procédé selon l'une quelconque des revendications 1 à 5, un ou plusieurs paramètres choisis dans le groupe constitué par la quantité totale de fluide supercritique par rapport au volume d'échantillon, le nombre de cycles de purge, la température pendant le contact avec le fluide supercritique, le taux d'échange avec le fluide supercritique, la température pendant l'étape d'évaporation, la vitesse de chauffage et la vitesse de la réduction de pression étant réglés.

7. Procédé selon l'une quelconque des revendications 1 à 6, ledit solvant organique polaire étant choisi dans le groupe constitué par l'acétone, l'acétonitrile, l'ammonique, le t-butanol, le n-propanol, l'éthanol, le méthanol et l'acide acétique.

8. Procédé selon l'une quelconque des revendications 2 à 7, lesdites molécules biologiques ou lesdits ions biologiques étant choisis dans le groupe constitué par les albumines, un lysozyme, les virus, les cellules, les γ-globulines d'origines humaine et animale, les immunoglobulines d'origine aussi bien humaine qu'animale, les protéines d'origine recombinante ou naturelle dont les polypeptides d'origine synthétique ou naturelle, l'interleukine-2 et son récepteur, les enzymes, les anticorps monoclonaux, les antigènes, les lectines, les protéines bactériennes de liaison aux immunoglobulines, la trypsine et son inhibiteur, le cytochrome C, la myoglobuline, une interleukine humaine recombinante, une protéine de fusion recombinante, la protéine A, la protéine G, la protéine L, le peptide H, les produits dérivés d'acides nucléiques, l'ADN d'origine soit synthétique soit naturelle, et l'ARN d'origine soit synthétique soit naturelle ;
éventuellement lesdites molécules biologiques ou lesdits ions biologiques étant la protéine A.

9. Procédé selon l'une quelconque des revendications 1 à 8, ledit élément support comprenant un matériau polymère choisi dans le groupe constitué par les polysulfones, les polyéthersulfones, les oxydes de polyphénylène, les polycarbonates, les polyesters, la cellulose et les dérivés de cellulose.

10. Procédé selon l'une quelconque des revendications 1 à 9, ledit matériau composite étant une membrane.

11. Procédé selon l'une quelconque des revendications 1 à 10, ledit matériau composite étant en contact avec une ou plusieurs couches intermédiaires.

12. Procédé selon la revendication 11, lesdits une ou plusieurs couches intermédiaires étant choisies dans le groupe constitué par un écran, le polypropylène, le polyéthylène et le papier.

13. Procédé selon l'une quelconque des revendications 1 à 12, ledit gel réticulé étant un hydrogel neutre, un hydrogel chargé, un gel polyélectrolytique, un gel hydrophobe, un gel neutre ou un gel comprenant des groupes fonctionnels.

14. Procédé selon l'une quelconque des revendications 1 à 13, ledit gel réticulé macroporeux comprenant des macropores comportant une taille moyenne d'environ 10 nm à environ 3000 nm.

15. Procédé selon l'une quelconque des revendications 1 à 14, lesdits pores de l'élément support comportant un diamètre moyen de pore d'environ 0,1 µm à environ 50 µm.
